# EUROPEAN PATENT SPECIFICATION

(11) **EP 3 357 416 B1**
(45) Date of publication and mention of the grant of the patent: **21.10.2020**
(21) Application number: 18155471.8
(22) Date of filing: 07.02.2018
(51) Int. Cl.: A61B 5/00

(54) **METHOD FOR PROVIDING SKIN INFORMATION AND ELECTRONIC DEVICE FOR SUPPORTING THE SAME**
VERFAHREN ZUR BEREITSTELLUNG VON HAUTINFORMATIONEN UND ELEKTRONISCHE VORRICHTUNG ZUR UNTERSTÜTZUNG DERSELBEN
PROCÉDÉ DE FOURNITURE D'INFORMATIONS SUR LA PEAU ET DISPOSITIF ÉLECTRONIQUE LE PRENANT EN CHARGE

(30) Priority: 07.02.2017 KR 20170017010
(43) Date of publication of application: 08.08.2018
(73) Proprietor: Samsung Electronics Co., Ltd., Gyeonggi-do 16677 (KR)
(72) Inventor: LEE, Dong Hyun, Gyeonggi-do, 17098 (KR); WON, Jin Hee, Gyeonggi-do, 15237 (KR); LEE, Jae Sung, Gyeonggi-do, 13532 (KR); CHOI, Jong Min, Seoul, 06001 (KR); KIM, Dong Wook, Seoul, 06589 (KR); KIM, Tae Ho, Chungcheongbuk-do, 28391 (KR); LEE, Seung Eun, Seoul, 06608 (KR)
(74) Representative: HGF

(56) References cited:
- WO-A1-2017/012675
- US-A1- 2014 378 810
- US-A1- 2016 125 228
- US-A1- 2016 261 793
- US-A1- 2016 296 119
- Jimmy Westenberg: "LG V10 officially announced: everything you need to know", , 30 September 2015 (2015-09-30), XP055481451, www.androidauthority.com Retrieved from the Internet: URL:https://web.archive.org/web/2016122215 5932/https://www.androidauthority.com/lg-v 10-specs-price-release-date-645871/ [retrieved on 2018-06-05]
- SEWOONG KIM ET AL: "Smartphone-based multispectral imaging: system development and potential for mobile skin diagnosis", BIOMEDICAL OPTICS EXPRESS, vol. 7, no. 12, 28 November 2016 (2016-11-28), page 5294, XP055481455, United States ISSN: 2156-7085, DOI: 10.1364/BOE.7.005294

## Description

### PRIORITY

This application claims priority to Korean Patent Application No. 10-2017-0017010, filed February 7, 2017.

### BACKGROUND OF THE INVENTION

### 1. Field of the Invention

The present disclosure relates to technologies of providing skin information about a user's body. More particularly, the present disclosure relates to an electronic device for capturing at least a portion of a user's body based on an infrared ray emitted from the electronic device and light emission by at least a partial region of a display, analyzing the captured image, and immediately providing user skin information.

### 2. Background of the Invention

As there has been an increase in interest or demand for skin management, various types of devices capable of measuring skin states of a portion of a user's body themselves to obtain skin information may have been proposed. However, a user may have a limit on accurate determination for his or her skin state or may be ignorant of proper correspondence information requested according to determined results. In this case, it may be less efficient in operating a skin measurement device, and the use of the skin measurement device may also be reduced correspondingly.

Thus, there is a need for a skin measurement device capable of more accurately diagnosing user skin states and easily obtaining related information.

The above information is presented as background information only to assist with an understanding of the present disclosure. No determination has been made, and no assertion is made, as to whether any of the above might be applicable as prior art with regard to the present disclosure.
US 2014/0378810 A1 discloses a method comprising capturing plural frames of skin imagery from a mammalian body, using a smartphone, deriving information from the captured imagery, and comparing the derived information with reference information and reporting results. Illumination at different spectral wavelengths may be provided by illumination from the smartphone screen and images may be captured using the smartphone's front-facing camera.
US 2016/0261793 A1 discloses a device comprising a first digital camera having a first center line of sight and a second digital camera having a second center line of sight that is parallel and opposing the first camera.

US 2016/0296119 A1 discloses an image analysis device, an image analysis method, a program, and an illumination device to efficiently acquire an image to be suitably used to analyze skin.

US 2016/0125228 A1 discloses a method for analyzing face information in an electronic device. It is disclosed that the method includes detecting at least one face region from an image that is being captured by a camera module, zooming in the at least one detected face region, and analyzing the at least one detected and zoomed in face region according to at least one analysis item.

### SUMMARY OF THE INVENTION

An aim of certain embodiments of the present disclosure is to provide an electronic device for capturing at least a portion of a user's body based on an infrared ray emitted from the electronic device and light emission by at least a partial region of a display, analyzing the captured image, and immediately providing user skin information.

In accordance with an aspect of the present disclosure, an electronic device is provided. The electronic device includes an image capture device comprising a source device configured to emit an infrared ray, a first camera, and a second camera configured to detect the infrared ray and to capture an image of at least a portion of a user's body based on the emitted infrared ray, a memory configured to store an image captured by the image capture device, a display configured to emit light in a specified color from at least one region based on driving at least one pixel, and at least one processor configured to be electrically connected with the image capture device, the memory, and the display, the at least one processor configured to: control to drive the display to emit the light in the specified color from the at least one region, control to drive the image capture device to obtain a first capture image by the first camera when the display emits the light in the specified color and a second capture image by the second camera when the light source device emits the infrared ray, generate a third image by performing registration of the first capture image and the second capture image, generate a spectrum for a light of a specific wavelength band corresponding to a specific pixel of the generated third image, and determine a skin state for one region of a user's body corresponding to the specific pixel based on a gradient and an area size of the generated spectrum.

In accordance with an aspect of the present disclosure, a method for providing skin information in an electronic device is provided. The method comprises: capturing an image of at least a portion of a user's body based on a light source device configured to emit an infrared ray, a first camera, and a second camera configured to detect the infrared ray; emitting light in a specified color from at least one region of a display of the electronic device based on driving at least one pixel of the display; controlling to drive the image capture device to obtain a first capture image by the first camera when the display emits the light in the specified color and a second capture image by the second camera when the light source device emits the infrared ray; generating a third image by performing registration of the first capture image and the second capture image, generating a spectrum for a light of a specific wavelength band corresponding to a specific pixel of the generated third image, and determining a skin state for one region of a user's body corresponding to the specific pixel based on a gradient and an area size of the generated spectrum.

According to an embodiment of the present disclosure, the at least one processor may be configured to control to drive the image capture device in a state where the at least one region of the display emits light in the specified color to obtain a first capture image by the first camera and a second capture image by the second camera and perform registration of the first capture image and the second capture image to generate a third image.

According to various embodiments of the present disclosure, the electronic device may obtain immediate, intuitive skin information about at least a portion of a user's body through a relatively simple operation of taking a selfie.

According to various embodiments of the present disclosure, the electronic device may construct a platform, such as a telemedicine service or a product recommendation service based on skin information by sharing the skin information of the user with at least one external device.

In addition, various effects directly or indirectly ascertained through the present disclosure may be provided.

Other aspects, advantages, and salient features of the disclosure will become apparent to those skilled in the art from the following detailed description, which, taken in conjunction with the annexed drawings, discloses various embodiments of the present disclosure.

### BRIEF DESCRIPTION OF THE DRAWINGS

The above and other aspects, features, and advantages of certain embodiments of the present disclosure will be more apparent from the following description taken in conjunction with the accompanying drawings, in which:
FIG. 1 illustrates an operation of an electronic device according to an embodiment of the present disclosure;
FIG. 2 is a block diagram illustrating a configuration of an electronic device according to an embodiment of the present disclosure;
FIG. 3 illustrates taking a selfie based on surface emission of an electronic device according to an embodiment of the present disclosure;
FIG. 4 illustrates a wave spectrum based on absorbance of a user's body according to an embodiment of the present disclosure;
FIG. 5A illustrates a first user interface incident to execution of a first application according to an embodiment of the present disclosure;
FIG. 5B illustrates a second user interface incident to execution of a second application according to an embodiment of the present disclosure;
FIG. 5C illustrates a third user interface incident to execution of a second application according to an embodiment of the present disclosure;
FIG. 6A is a flowchart illustrating a method for providing skin information in an electronic device according to an embodiment of the present disclosure;
FIG. 6B illustrates a service platform associated with providing skin information in an electronic device according to an embodiment of the present disclosure;
FIG. 7 illustrates an electronic device in a network environment according to an embodiment of the present disclosure;
FIG. 8 is a block diagram illustrating an electronic device according to an embodiment of the present disclosure; and
FIG. 9 is a block diagram illustrating a program module according to an embodiment of the present disclosure.

Throughout the drawings, it should be noted that like reference numbers are used to depict the same or similar elements, features, and structures.

### DETAILED DESCRIPTION OF EMBODIMENTS OF THE PRESENT INVENTION

The following description with reference to the accompanying drawings is provided to assist in a comprehensive understanding of various embodiments of the present disclosure as defined by the claims and their equivalents. It includes various specific details to assist in that understanding but these are to be regarded as merely exemplary. Accordingly, those of ordinary skill in the art will recognize that various changes and modifications of the various embodiments described herein can be made without departing from the scope of the present disclosure. In addition, descriptions of well-known functions and constructions may be omitted for clarity and conciseness.

The terms and words used in the following description and claims are not limited to the bibliographical meanings, but, are merely used by the inventor to enable a clear and consistent understanding of the present disclosure. Accordingly, it should be apparent to those skilled in the art that the following description of various embodiments of the present disclosure is provided for illustration purpose only and not for the purpose of limiting the present disclosure as defined by the appended claims and their equivalents.

It is to be understood that the singular forms "a," "an," and "the" include plural referents unless the context clearly dictates otherwise. Thus, for example, reference to "a component surface" includes reference to one or more of such surfaces.

By the term "substantially" it is meant that the recited characteristic, parameter, or value need not be achieved exactly, but that deviations or variations, including for example, tolerances, measurement error, measurement accuracy limitations and other factors known to those of skill in the art, may occur in amounts that do not preclude the effect the characteristic was intended to provide.

The term "include," "comprise," and "have", or "may include," or "may comprise" and "may have" used herein indicates disclosed functions, operations, or existence of elements but does not exclude other functions, operations or elements.

For example, the expressions "A or B," or "at least one of A and/or B" may indicate A and B, A, or B. For instance, the expression "A or B" or "at least one of A and/or B" may indicate (1) at least one A, (2) at least one B, or (3) both at least one A and at least one B.

The terms, such as "1st," "2nd," "first," "second," and the like used herein may refer to modifying various different elements of various embodiments of the present disclosure, but are not intended to limit the elements. For instance, "a first user device" and "a second user device" may indicate different user devices regardless of order or importance. For example, a first component may be referred to as a second component and vice versa without departing from the scope of the present disclosure.

In various embodiments of the present disclosure, it is intended that when a component (for example, a first component) is referred to as being "operatively or communicatively coupled with/to" or "connected to" another component (for example, a second component), the component may be directly connected to the other component or connected through another component (for example, a third component). In various embodiments of the present disclosure, it is intended that when a component (for example, a first component) is referred to as being "directly connected to" or "directly accessed" another component (for example, a second component), another component (for example, a third component) does not exist between the component (for example, the first component) and the other component (for example, the second component).

The expression "configured to" used in various embodiments of the present disclosure may be interchangeably used with "suitable for," "having the capacity to," "designed to," "adapted to," "made to," or "capable of' according to the situation, for example. The term "configured to" may not necessarily indicate "specifically designed to" in terms of hardware. Instead, the expression "a device configured to" in some situations may indicate that the device and another device or part are "capable of." For example, the expression "a processor configured to perform A, B, and C" may indicate a dedicated processor (for example, an embedded processor) for performing a corresponding operation or a general purpose processor (for example, a central processing unit (CPU) or application processor (AP)) for performing corresponding operations by executing at least one software program stored in a memory device.

Terms used in various embodiments of the present disclosure are used to describe certain embodiments of the present disclosure, but are not intended to limit the scope of other embodiments. The terms of a singular form may include plural forms unless they have a clearly different meaning in the context. Otherwise, all terms used herein may have the same meanings that are generally understood by a person skilled in the art. In general, terms defined in a dictionary should be considered to have the same meanings as the contextual meaning of the related art, and, unless clearly defined herein, should not be understood differently or as having an excessively formal meaning. In any case, even the terms defined in the present specification are not intended to be interpreted as excluding embodiments of the present disclosure.

An electronic device according to various embodiments of the present disclosure may include at least one of a smartphone, a tablet personal computer (PC), a mobile phone, a video telephone, an electronic book reader, a desktop PC, a laptop PC, a netbook computer, a workstation, a server, a personal digital assistant (PDA), a portable multimedia player (PMP), a moving picture experts group phase 1 or phase 2 (MPEG-1 or MPEG-2) audio layer 3 (MP3) player, a mobile medical device, a camera, or a wearable device. The wearable device may include at least one of an accessory-type device (e.g., a watch, a ring, a bracelet, an anklet, a necklace, glasses, a contact lens, a head-mounted device (HMD)), a textile- or clothing-integrated-type device (e.g., an electronic apparel), a body-attached-type device (e.g., a skin pad or a tattoo), or a bio-implantable-type device (e.g., an implantable circuit).

In some various embodiments of the present disclosure, an electronic device may be a home appliance. The smart home appliance may include at least one of, for example, a television (TV), a digital video/versatile disc (DVD) player, an audio, a refrigerator, an air conditioner, a cleaner, an oven, a microwave oven, a washing machine, an air cleaner, a set-top box, a home automation control panel, a security control panel, a TV box (e.g., Samsung HomeSync™, Apple TV™, or Google TV™), a game console (e.g., Xbox™ or PlayStation™), an electronic dictionary, an electronic key, a camcorder, or an electronic picture frame.

In other various embodiments of the present disclosure, an electronic device may include at least one of various medical devices (e.g., various portable medical measurement devices (e.g., a blood glucose measuring device, a heart rate measuring device, a blood pressure measuring device, a body temperature measuring device, or the like), a magnetic resonance angiography (MRA), a magnetic resonance imaging (MRI), a computed tomography (CT), a scanner, an ultrasonic device, or the like), a navigation device, a global navigation satellite system (GNSS), an event data recorder (EDR), a flight data recorder (FDR), a vehicle infotainment device, electronic equipment for vessels (e.g., a navigation system, a gyrocompass, or the like), avionics, a security device, a head unit for a vehicle, an industrial or home robot, an automatic teller machine (ATM), a point of sales (POS) device of a store, or an Internet of things (IoT) device (e.g., a light bulb, various sensors, an electric or gas meter, a sprinkler, a fire alarm, a thermostat, a streetlamp, a toaster, exercise equipment, a hot water tank, a heater, a boiler, or the like).

According to various embodiments of the present disclosure, an electronic device may include at least one of a part of furniture or a building/structure, an electronic board, an electronic signature receiving device, a projector, or a measuring instrument (e.g., a water meter, an electricity meter, a gas meter, a wave meter, or the like). An electronic device may be one or more combinations of the above-mentioned devices. An electronic device according to some various embodiments of the present disclosure may be a flexible device. An electronic device according to an embodiment of the present disclosure is not limited to the above-mentioned devices, and may include new electronic devices with the development of new technology.

Hereinafter, an electronic device according to various embodiments of the present disclosure will be described with reference to the accompanying drawings. The term "user" used herein may refer to a person who uses an electronic device or may refer to a device (e.g., an artificial intelligence electronic device) that uses an electronic device.

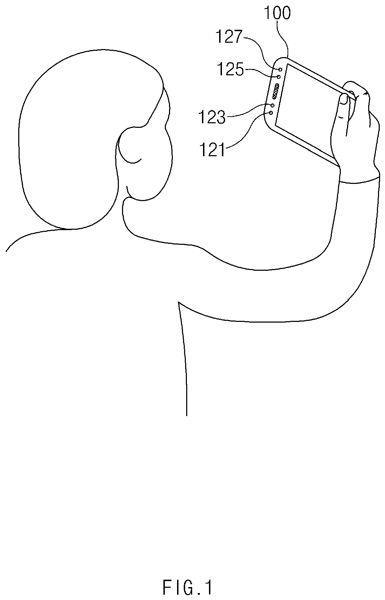

FIG. 1 illustrates an operation of an electronic device according to an embodiment of the present disclosure.

Referring to FIG. 1, an electronic device 100 may mount at least one of a light source module 121, a sensor module 123, a first camera 125 (e.g., a front camera), or a second camera 127 (e.g., an iris camera) on at least a partial region of its front surface. The light source module 121, the sensor module 123, the first camera 125, and/or the second camera 127 may be located to be spaced apart from each other at a specified distance on the electronic device 100.

In an embodiment of the present disclosure, the light source module 121 may include at least one of an organic light emitting diode (OLED) or a laser diode (LD) which emits an electromagnetic wave (e.g., an infrared ray) of a specified wavelength band. The sensor module 123 may include at least one of, for example, an illumination sensor or a proximity sensor and may sense at least one of brightness for a region around the electronic device 100 or proximity of an object, thus transmitting information about the sensed result to a controller (or a processor of the electronic device 100). The first camera 125 may capture a video or an image for a first direction (e.g., a front of the electronic device 100) with respect to the electronic device 100. If an electromagnetic wave emitted from the light source module 121 is reflected from any object (or subject), the second camera 127 may detect or receive the reflected electromagnetic wave.

In an embodiment of the present disclosure, if a specified image capture mode is selected by a user on an execution screen where a specified application (e.g., a camera application) is executed, the electronic device 100 may perform a specific operation based on at least one of the light source module 121, the first camera 125, or the second camera 127. For example, the electronic device 100 may take a selfie of the user (e.g., capture an image for at least a portion of a user's body) based on at least one of the light source module 121, the first camera 125, or the second camera 127. In this operation, the electronic device 100 may analyze a skin state of the user based on the selfie image and may provide the analyzed information.

In an embodiment of the present disclosure, activation of the specified image capture mode may entail controlling a function for another element of the electronic device 100. For example, if the specified image capture mode is activated, a user interface (or another execution screen where the specified application is executed) capable of controlling a surface emission function of a display may be output. For example, the surface emission may mean that a plurality of pixels corresponding to a screen region of the display emit light with a light of a specified wavelength band during a specified time based on user control or specified scheduling information. A user may provide an input (or a touch) to an object (e.g., a selection window, a tap button, or the like) displayed on the user interface to set a light of a specific wavelength band associated with surface emission of the display. For example, the user may set the display such that the display performs surface emission with a light of a first wavelength band (e.g., a light of a red wavelength band, a light of a green wavelength band, or a light of a blue wavelength band) or may set the display such that at least two of lights of first to third wavelength bands (e.g., a light of a red wavelength band, a light of a green wavelength band, and a light of a blue wavelength band) have a specified time difference (e.g., a time difference within a range of 0.5 seconds to 1 second) and perform surface emission. Alternatively, the user may set the display to emit a light of a fourth wavelength band (e.g., a light of a white wavelength band) in which the lights of the first to third wavelength bands are mixed. If the display performs surface emission with the light of the fourth wavelength band, for example, the electronic device 100 may extract only a light of a specific wavelength band from light received using a color filter included in the electronic device 100.

In an embodiment of the present disclosure, if the setting of the surface emission for the display is completed by the user, the first camera 125 may be driven and at least a portion of a user's body may be displayed on a screen region of the display. In this operation, an object (or a contour) which functions as a focus guide for a specific region (e.g., a face) of the user's body may be displayed on the screen region of the display. Alternatively, if a distance between the first camera 125 and the user's body is greater than a specified distance range, the electronic device 100 may output feedback (e.g., vibration, a notification sound, a message, or the like) which functions as a notification. Thereafter, if a user input for instructing to perform image capture is provided to an input interface (e.g., a physical key button) on the electronic device 100 or at a time when a timer set in connection with image capture elapses, the display may perform surface emission with a light of a specific wavelength band set by the user, and a screen region may be displayed in a color of the light.

According to the surface emission of the display, at least one of the light source module 121, the first camera 125, or the second camera 127 may start a corresponding function operation. For example, the light source module 121 may emit an infrared ray in a specified direction (e.g., a front of the electronic device 100). The second camera 127 may receive an infrared ray reflected from one region of the user's body located in front of the electronic device 100 and may generate a first image based on the infrared ray. Further, the first camera 125 may receive a light of a specific wavelength band reflected from the user depending on surface emission of the display based on the light of the specific wavelength band and may generate a second image. In an embodiment of the present disclosure, if a light of a specific wavelength band due to surface emission of the display is set to a plurality of lights, the second image may be generated as a plurality of images in response to a plurality of numbers of surface emission by each light of the specific wavelength band.

The generated first image and the generated second image may be stored in a specified region (e.g., a memory) on the electronic device 100. In an embodiment of the present disclosure, the first image and the second image may be generated at the same or similar time or may be generated at a time corresponding to a specified operation order among the light source module 121, the first camera 125, and the second camera 127, which cause generation of each image.

If image capture by the first camera 125 and the second camera 127 is ended, surface emission of the display may be released. Alternatively, if a light of a specific wavelength band is set to a plurality of lights in connection with the surface emission of the display and if first image capture of the first camera 125 and the second camera 127 according to first surface emission is ended, the first surface emission may be released and second surface emission is started after pausing during a specified time (e.g., a set time difference between the plurality of lights). The first camera 125 and the second camera 127 may perform second image capture depending on the start of the second surface emission.

In an embodiment of the present disclosure, a controller (or a processor) of the electronic device 100 may generate a separate image (hereinafter referred to as "registration image") by performing registration of a plurality of capture images by the first camera 125 and the second camera 127. The controller may perform a series of analysis processes for the registration image to obtain skin information about at least one captured region of a user's body. The controller may store the skin information in a memory region, and may transmit the stored skin information to an external device which constructs a network with the electronic device 100 or may output the stored skin information on a screen region of the display.

Hereinafter, a description will be given of various embodiments associated with obtaining or providing the skin information and function operations of elements of the electronic device 100 which supports the various embodiments.

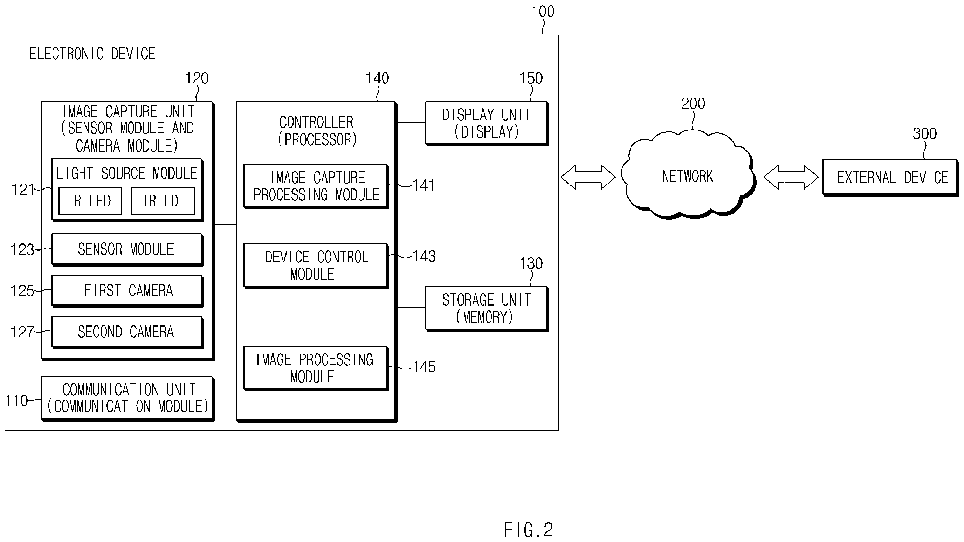

FIG. 2 is a block diagram illustrating a configuration of an electronic device according to an embodiment of the present disclosure.

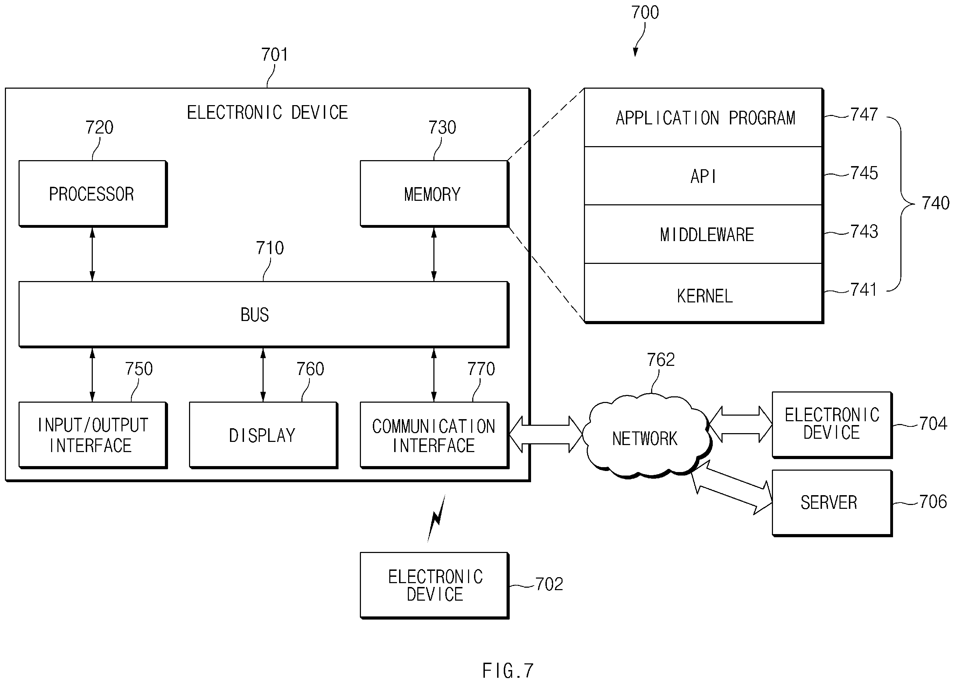

Referring to FIG. 2, an electronic device 100 may include a communication unit 110 (or a communication module), an image capture unit 120 (or an image capture module, or a sensor module and a camera module), a storage unit 130 (or a memory), a controller 140 (or a processor), and a display unit 150 (or a display). In various embodiments of the present disclosure, the electronic device 100 may exclude at least one of the above-mentioned elements or may further include another element. For example, the electronic device 100 may further include a power supply device (e.g., a battery) for supplying a driving power to the elements or elements of an electronic device 701 which will be described below with reference to FIG. 7.

The communication unit 110 may construct a network 200 with at least one external device 300 and may be electrically or communicatively connected with the at least one external device 300. The communication unit 110 may establish wired communication or wireless communication complying with a defined protocol with the at least one external device 300 and may access the network 200 based on the wired communication or the wireless communication, thus transmitting skin information of a user to the external device 300. For example, as described above, the communication unit 110 may transmit skin information about at least a portion of a user's body, obtained through a selfie image, to the external device 300 associated with at least one of a medical institution, a cosmetics company (or a beauty company), or an online/offline shopping mall company. In this operation, the communication unit 110 may transmit only at least partial skin information in response to user control. Further, the communication unit 110 may transmit, for example, information about an avatar to which the skin information generated by the controller 140 is mapped to the external device 300. In an embodiment of the present disclosure, the communication unit 110 may receive feedback information, recommended beauty product information, or the like corresponding to the skin information from the external device 300.

The image capture unit 120 may capture a region around the electronic device 100 or an object adjacent to the electronic device 100 in response to user control or specified scheduling information. For example, the image capture unit 120 may take a selfie of a user located in front of the electronic device 100 to generate a capture image for at least one region of a user's body. In this regard, the image capture unit 120 may include at least one of a light source module 121 (e.g., including at least one of an infrared (IR) LED or an IR LD), a sensor module 123 (e.g., including an illumination sensor), a first camera 125 (e.g., a front camera), or a second camera 127 (e.g., an iris camera). In an embodiment of the present disclosure, at least a portion of the light source module 121, the first camera 125, or the second camera 127 may start to be driven according to activation of a specified image capture mode selected by a user on a related application (e.g., a camera application). Function operations of the elements are described above with reference to FIG. 1, and a repeated description may be omitted.

In various embodiments of the present disclosure, the image capture unit 120 may further include a third camera (e.g., a rear surface) other than the elements. The first camera 125, the second camera 127, and the third camera may be located on the electronic device 100 to capture different regions or at least a partially overlapped region. For example, the first camera 125 and/or the second camera 127 may be located on one region of a front surface of the electronic device 100, and the third camera may be located on one region of a rear surface of the electronic device 100.

The storage unit 130 may store an instruction or data associated with function operations of the electronic device 100. For example, the storage unit 130 may store at least one image generated by the image capture unit 120 and analysis information associated with the image or may store at least one information (e.g., feedback information, recommended beauty product information, or the like corresponding to skin information) received through interaction with the external device 300. Further, the storage unit 130 may store an application program (e.g., a camera application) for controlling to drive the image capture unit 120 or at least one application program (e.g., a gallery application, a health management service application, or the like) for supporting to display the image and skin information in response to user control. In an embodiment of the present disclosure, a mapping database or index between a light (or signal) of a specific wavelength band and an image color according to the light may be constructed in the storage unit 130 in connection with surface emission of the above-mentioned display unit 150. In various embodiments of the present disclosure, the storage unit 130 may include a secure region (e.g., a trust zone) accessible based on a specified signal or route. The storage unit 130 may store, for example, user personal information, such as an image generated by taking a selfie in the secure region.

The controller 140 may be electrically or operatively connected with other elements of the electronic device 100 and may perform control, communication, an arithmetic operation, or data processing for the elements. For example, the controller 140 may perform registration of images captured by the first camera 125 and the second camera 127 based on image processing and may analyze the registration image, thus providing related skin information. In an embodiment of the present disclosure, in connection with processing a series of processes incidental to providing the skin information, the controller 140 may include at least one of an image capture processing module 141, a device control module 143, or an image processing module 145.

The image capture processing module 141 may detect, for example, a selection event of the user for a specified image capture mode, generated on an execution screen where a specified application (e.g., a camera application) is executed and may output a user interface capable of setting a surface emission function of the display unit 150. The device control module 143 may control to drive at least one of the light source module 121, the first camera 125, or the second camera 127 in response to the completion of the setting of the surface emission function. The image processing module 145 may generate a registration image by performing registration of a plurality of images by the first camera 125 and the second camera 127. In an embodiment of the present disclosure, the image processing module 145 may generate the registration image based on feature point mapping between the plurality of images. In this regard, the image processing module 145 may extract, for example, a contour for at least one region of a user's body on each image based on image processing, such as an edge detection filter or an active contours model. The image processing module 145 may identify at least one feature point in an inner region of the contour and may generate the registration image by performing registration of the plurality of images such that feature points corresponding to each other on each image are identical to each other.

In an embodiment of the present disclosure, the image processing module 145 may determine a light of a specific wavelength band corresponding to a color of each pixel of the registration image with reference to a database or index constructed in the storage unit 130. The image processing module 145 may determine a skin state of the user based on an image color differently indicated on each pixel of a registration image depending on an absorbance degree of a user's body when capturing a user based on surface emission of the display unit 150 and infrared ray emission of the light source module 121. In an embodiment of the present disclosure, the image capture processing module 141 may output information associated with the determined skin state through an execution screen where a specified application (e.g., a gallery application or a health management service application) is executed or a specified user interface. If a user input (e.g., a touch) is provided to a specified region in the execution screen or the user interface, the device control module 143 may control at least one of the communication unit 110 or the storage unit 130 to transmit information on the execution screen or the user interface to the external device 300.

The display unit 150 may display a variety of content (e.g., a text, an image, a video, an icon, a symbol, or the like). For example, the display unit 150 may output an image generated by an image capture operation of the first camera 125 or the second camera 127 in the form of a preview. Alternatively, the display unit 150 may output an execution screen where an application is executed, in response to user control or specified scheduling information and may display analysis information (e.g., skin information) about the image on the execution screen. In various embodiments of the present disclosure, if specific information (e.g., medical treatment information, beauty product information, or the like associated with a skin) is received from the external device 300 via the communication unit 110, the display unit 150 may output content including the specific information.

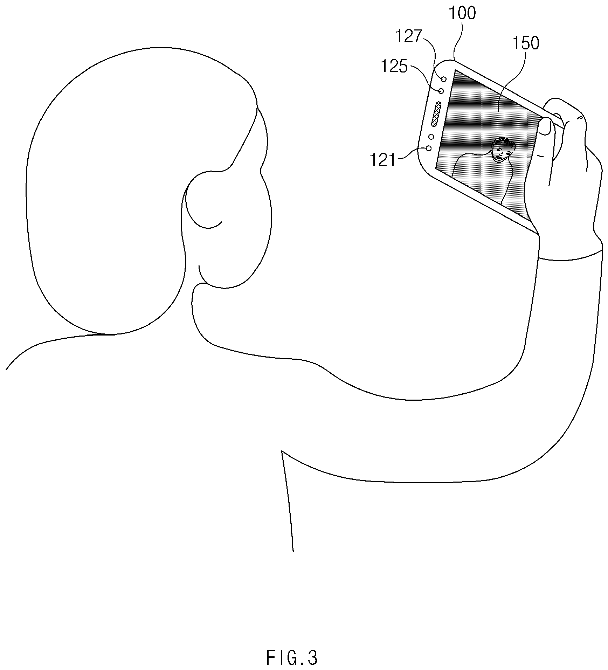

FIG. 3 illustrates taking a selfie based on surface emission of an electronic device according to an embodiment of the present disclosure.

Referring to FIG. 3, surface emission performance of a display unit 150 and a function operation of at least one of a light source module 121, a first camera 125, or a second camera 127 may be triggered in response to activation of a specified image capture mode selected by a user on an execution screen when a specified application (e.g., a camera application) is executed. In this regard, at least one object (e.g., a tap button) associated with setting an image capture mode of an image capture unit 120 of FIG. 2 may be included on the execution screen where the application is executed. For example, an object associated with activating at least one of a beauty image capture mode, a panorama image capture mode, or a continuous image capture mode may be included on the execution screen. In an embodiment of the present disclosure, if the beauty image capture mode (e.g., a mode of providing skin state information through image capture) is activated by user control, the execution screen may be converted into another execution screen or a user interface associated with setting surface emission of the display unit 150. Alternatively, if an image capture mode (e.g., the panorama image capture mode or the continuous image capture mode) except for the beauty image capture mode is activated, image capture may be performed by at least one of the first camera 125 or a third camera (e.g., a rear camera). In an embodiment of the present disclosure, if a setting of surface emission is completed on the converted other execution screen or the converted user interface, at least a portion of a user's body may be displayed on a screen region of the display unit 150 by driving of the first camera 125.

In this regard, referring to FIG. 3, if a user instructs to take a selfie (e.g., if he or she provides an input to an input interface associated with image capture of a camera), the display unit 150 may perform surface emission by control of a controller 140 of FIG. 2. In this operation, the controller 140 may control a plurality of pixels included in the display unit 150 based on a light (or an optical signal) of a specific wavelength band selected by the user in connection with setting surface emission. For example, the controller 140 may supply voltage corresponding to a gray scale of the light of the selected specific wavelength band to at least one device (e.g., a red, green, blue (RGB) device) included in each of the plurality of pixels of the display unit 150 to control light emission of the device. In various embodiments of the present disclosure, the controller 140 may further perform pixel control of the display unit 150 for a light of a separate wavelength band (e.g., a light of a white wavelength band) (hereinafter referred to as "second light") other than the light of the specific wavelength band (hereinafter referred to as "first light") selected by the user. In this regard, the controller 140 may exclude an ambient light which interferes in the light of the wavelength band (e.g., the first light) selected by the user when an image capture unit 120 of FIG. 2 is driven, by referring to a difference image with respect to capture images by each of surface emission corresponding to the first light and surface emission corresponding to the second light. Alternatively, the controller 140 may exclude interference of an ambient light with reference to a capture image according to surface emission based on the first light and a difference image between images captured in a state where the plurality of pixels of the display unit 150 are not emitted.

As such, if the plurality of pixels of the display unit 150 are controlled by the controller 140, a screen region of the display unit 150 may be displayed in a color corresponding to a light of a specific wavelength band depending on light emission of at least one device included in each of the plurality of pixels. In this case, at least one of the light source module 121, the first camera 125, or the second camera 127 of the image capture unit 120 may start a function operation and may capture at least a portion of a user's body to generate a first image based on an infrared ray and/or a second image based on surface emission.

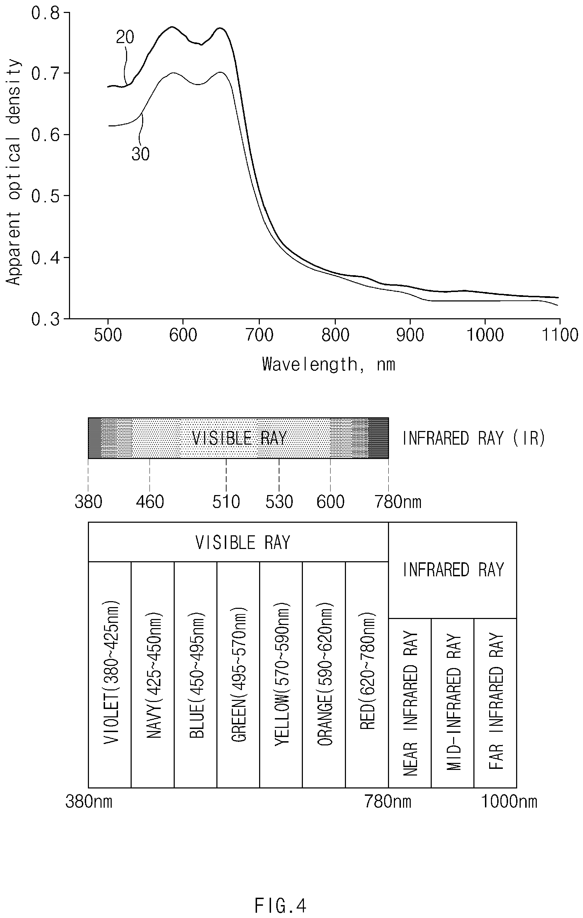

FIG. 4 illustrates a wave spectrum based on absorbance of a user's body according to an embodiment of the present disclosure.

Referring to FIG. 4, according to an embodiment of the present disclosure, a controller 140 of FIG. 2 may generate a registration image by performing registration of a plurality of images captured by a first camera 125 and a second camera 127 of FIG. 2 and may determine a skin state of a user based on the registration image. In this regard, a color corresponding to each of the plurality of pixels on the registration image may vary according to a degree to which a light of a specific wavelength band according to surface emission of a display unit 150 of FIG. 2 is absorbed to a subject (or a portion of a user's body). The controller 140 may determine a light of a specific wavelength band corresponding to a color for each of the plurality of pixels of the registration image with reference to a mapping database or index between a light (or signal) of a specific wavelength band and an image color, constructed in a storage unit 130 of FIG. 2. The controller 140 may generate a wave spectrum for the determined light of the specific wavelength band corresponding to each of the plurality of pixels of the registration image.

Referring to FIG. 4, as a color for each of the plurality of pixels of the registration image (or a light of a specific wavelength band corresponding to each of the plurality of pixels) varies, a first wave spectrum 20 for a first pixel, generated by the controller 140, and a second wave spectrum 30 for a second pixel, generated by the controller 140, may have different gradients or areas. In an embodiment of the present disclosure, the gradient may refer to a melanin index. It may be determined that a region of a user's body corresponding to a corresponding pixel on a registration image has a higher melanin index as the gradient is sharper. Further, the area may refer to an erythema index. It may be determined that a region of a user's body corresponding to a corresponding pixel on a registration image has a higher erythema level as the area is wider. As such, the controller 140 may identify a light of a specific wavelength band corresponding to a color of a corresponding pixel with respect to each pixel of the registration image and may analyze a wave spectrum for the identified light of the specific wavelength band, thus obtaining information of a melanin index or an erythema level for a region of a user's body corresponding to each pixel. In an embodiment of the present disclosure, the controller 140 may compare a color for each pixel of the registration image with a specified skin tone index and may collect skin tone information for each pixel (e.g., average a plurality of skin tone information or extract weighty information among the plurality of skin tone information) to obtain skin tone information about one region of a user's body.

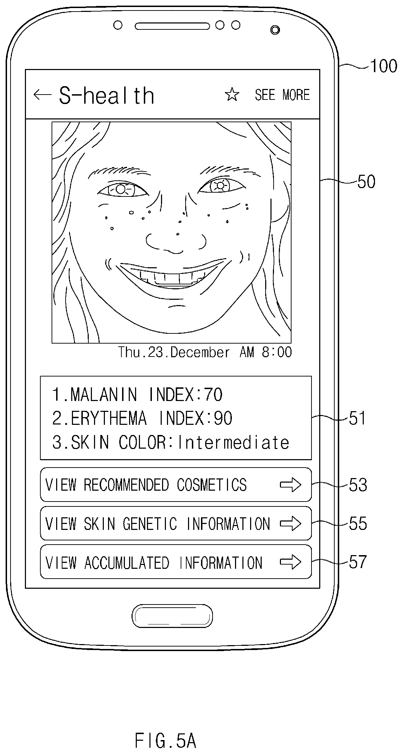

FIG. 5A illustrates a first user interface incident to execution of a first application according to an embodiment of the present disclosure.

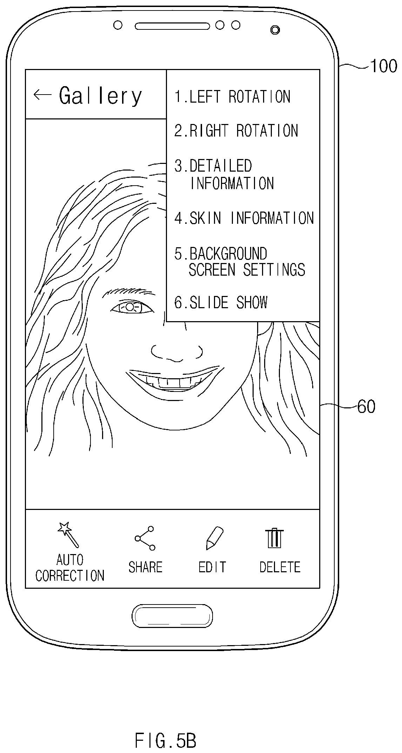

FIG. 5B illustrates a second user interface incident to execution of a second application according to an embodiment of the present disclosure.

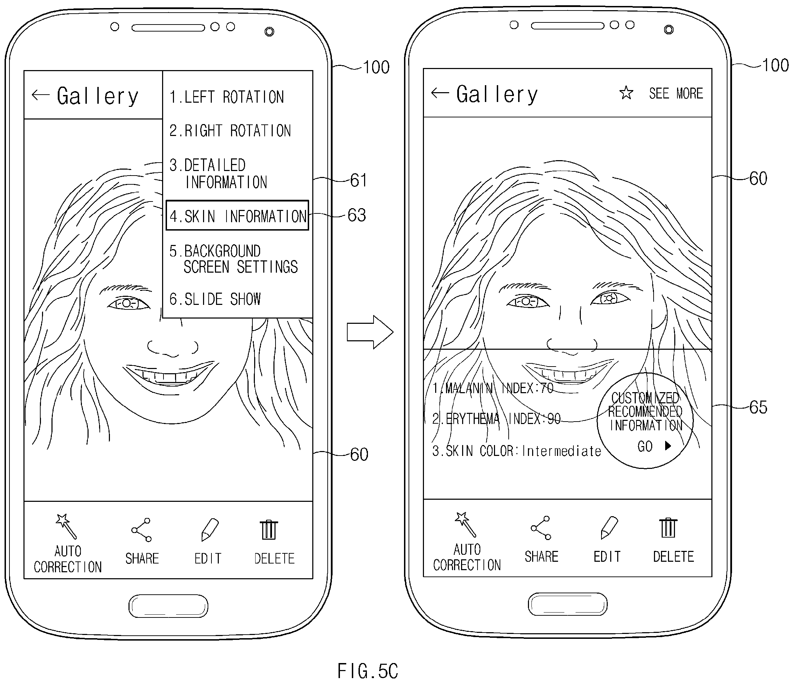

FIG. 5C illustrates a third user interface incident to execution of a second application according to an embodiment of the present disclosure.

In an embodiment of the present disclosure, correction (e.g., a whitening effect, a blemish removal effect, or the like) for at least one region of an image captured based on surface emission of a display unit 150 of FIG. 2 may be performed by an image processing module 145 of a controller 140 of FIG. 2. Display (or output) of each of an image based on the surface emission and the correction image may be supported through an operation of a specified first application (e.g., a gallery application). For example, a first object for supporting to output an image based on the surface emission through conversion (or interworking) into a second application different from the first application and a second object for supporting to output the correction image through another execution screen where the first application is executed may be included on an execution screen where the first application is executed.

Referring to FIG. 5A, if a user input (e.g., a touch) is provided on the first object, the execution screen where the first application is executed may be converted into an execution screen 50 where a separate second application (e.g., a health management service application (S-health)) is executed. An image based on surface emission for at least a portion of a user's body may be included on the execution screen 50 where the second application is executed. In this regard, a capture image based on the surface emission may be user personal information and may be stored in a secure region of a storage unit 130 of FIG. 2. Thus, in various embodiments of the present disclosure, when the first application is converted into the second application (or when a user input is provided on the first object), the controller 140 may perform specified user authentication (e.g., iris authentication, fingerprint authentication, pattern authentication, or password authentication) and may output the execution screen 50 where the second application is executed, only if the user authentication is met.

In an embodiment of the present disclosure, if a user is provided on one region (e.g., a cheek, a forehead, a nose, a chin, or the like) of an image based on surface emission, displayed on the execution screen 50, during a specified time or more, a third object 51 including skin information about the region may be displayed. In this operation, the controller 140 may identify a pixel corresponding to a user input region provided on the image based on the surface emission among a plurality of pixels included in the above-mentioned registration image and may read skin information of the identified pixel from the storage unit 130 to generate the third object 51. In various embodiments of the present disclosure, a thumbnail of a previously generated registration image may be displayed together with the third object 51. A user may face a change in skin from the past through the image based on the surface emission and the thumbnail.

In an embodiment of the present disclosure, at least one object 53, 55, and/or 57 for supporting to display or output a variety of information associated with skin information included in the third object 51 may be further displayed on the execution screen 50 where the second application is executed. If a user input is provided on the fourth object 53, the controller 140 may read and display cosmetics information corresponding to user skin information, which is received from an external device 300 of FIG. 2 (e.g., a cosmetics company server, a shopping mall company server, or the like) and is stored in the storage unit 130. Alternatively, in various embodiments of the present disclosure, cosmetics information corresponding to various skin states may be constructed as a database in the storage unit 130 of the electronic device 100. If a user input is provided on the fourth object 53, the controller 140 may obtain and display cosmetics information corresponding to the user on the cosmetics database.

According to an embodiment of the present disclosure, providing a user input on the fifth object 55 may be to output comparison analysis information associated with comparing and analyzing genetic information of a user, provided from the specified external device 300 (e.g., a medical institution server) with skin information of the user, determined through a registration image. The comparison analysis information may include, for example, skin trouble information vulnerable to the genetic information of the user, skin management information about the skin trouble information, and the like. In an embodiment of the present disclosure, the user may consider setting surface emission when he or she takes a selfie for measuring a skin state later, based on comparison analysis information output through the fifth object 55. For example, the user may consider selecting a light of a specific wavelength band associated with setting surface emission, sequentially setting surface emission for lights of a plurality of wavelength bands, or combining the lights of the plurality of wavelength bands to measure a skin trouble vulnerable to genetic information of the user. Alternatively, when making an appointment with a medical institution later based on the comparison analysis information, the user may reflect a concerned or interested item. According to an embodiment of the present disclosure, the sixth object 57 may support to display accumulated data for a skin state of the user. The user may monitor a numeric value for a change in skin state with reference to the accumulated data. In an embodiment of the present disclosure, information displayed by the fifth object 55 or the sixth object 57 may be transmitted to the external device 300 (e.g., the medical institution server, the shopping mall company server, or the cosmetics company server) in response to user control. Thus, the user may request an institution or company which operates the external device 300 to provide a questionnaire, recommend a cosmetics product, or provide feedback on a skin state.

Referring to FIGS. 5B and 5C, the user may provide an input (e.g., a touch) on a second object for supporting to output the correction image on the execution screen where the first application (e.g., a gallery application) is executed. In this case, the execution screen where the first application is executed may be converted into another execution screen 60 including a correction image of the first application. The user may perform additional correction for a displayed correction image through a first menu (e.g., an "edit" menu) included on the other execution screen 60. For example, the user may perform correction of removing erythema or melanic pigment or correction of changing a skin tone, based on user skin information provided from the electronic device 100. Alternatively, the user may transmit or upload the correction image to an external device (or an external server) (e.g., a social network service server or the like) through a second menu (e.g., a "share" menu) included in the other execution screen 60.

In an embodiment of the present disclosure, the other execution screen 60 may include a third menu (e.g., a "see more" menu) for supporting to operate a displayed correction image in various manners. If a user input is provided on the third menu, a specified menu window 61 (e.g., a menu window for supporting at least one of left rotation, right rotation, detailed information, skin information, background screen settings, or a slide show) may be displayed. In an embodiment of the present disclosure, if the user selects a skin information category 63 on the specified menu window 61 and provides an input (e.g., a touch) to one region of the correction image, an interface 65 including skin information about a region of the user input may be output on at least one region of the other execution screen 60. In an embodiment of the present disclosure, the interface 65 including the skin information may be represented as a specified display effect (e.g., a semi-transparent effect) based on visibility of a displayed correction image.

An electronic device according to various embodiments may include an image capture device comprising a light source device configured to emit an electromagnetic wave of a specified wavelength band, a first camera, and a second camera configured to detect the electromagnetic wave and to capture an image of at least a portion of a user's body based on the emitted electromagnetic wave, a memory configured to store an image captured by the image capture device, a display configured to emit light in a specified color from at least one region based on driving at least one pixel, and at least one processor electrically connected with the image capture device, the memory, and the display.

According to various embodiments of the present disclosure, the at least one processor may control to drive the display to emit the light in the specified color from the at least one region, control to drive the image capture device to obtain a first capture image by the first camera and a second capture image by the second camera when the display emits the light in the specified color, and may perform registration of the first capture image and the second capture image to generate a third image.

According to various embodiments of the present disclosure, the at least one processor may construct a database for an image color corresponding to a light of a specific wavelength band and may determine a light of a specific wavelength band corresponding to a color of each pixel of the third image with reference to the database.

According to various embodiments of the present disclosure, the at least one processor may generate a spectrum for a light of a specific wavelength band corresponding to a specific pixel of the third image and may determine a skin state for one region of a user's body corresponding to the specific pixel based on the spectrum.

According to various embodiments of the present disclosure, the at least one processor may determine a melanin index for the one region of the user's body based on a gradient of the spectrum and may determine an erythema index for the one region of the user's body based on an area of the spectrum.

According to various embodiments of the present disclosure, the at least one processor may collect color information about each pixel of the third image and may determine a skin tone for one region of a user's body corresponding to the third image.

According to various embodiments of the present disclosure, the at least one processor may output a first user interface including the first capture image or a correction image in which a specified effect is assigned to the first capture image.

According to various embodiments of the present disclosure, if a user input event on one region of the first capture image or the correction image occurs, the at least one processor may identify a pixel corresponding to a user input region on the first capture image or the correction image among at least one pixel of the third image and may output skin information about one region of a user's body corresponding to the identified pixel.

According to various embodiments of the present disclosure, the electronic device may further include a communication device configured to communicate with at least one specified external device.

According to various embodiments of the present disclosure, the at least one processor may control to drive the communication device to transmit the skin information to the at least one specified external device and may control to drive the communication device to receive at least one information corresponding to the skin information from the at least one specified external device.

According to various embodiments of the present disclosure, if an event of activating a specified image capture mode associated with determining a skin state through image capture occurs, the at least one processor may output a second user interface capable of setting a light of at least one specific wavelength band in connection with light emission of the display.

According to various embodiments of the present disclosure, if the at least one region of the display emits light with the light of the specified wavelength band, the at least one processor may control to drive at least one of the light source device, the first camera, and the second camera.

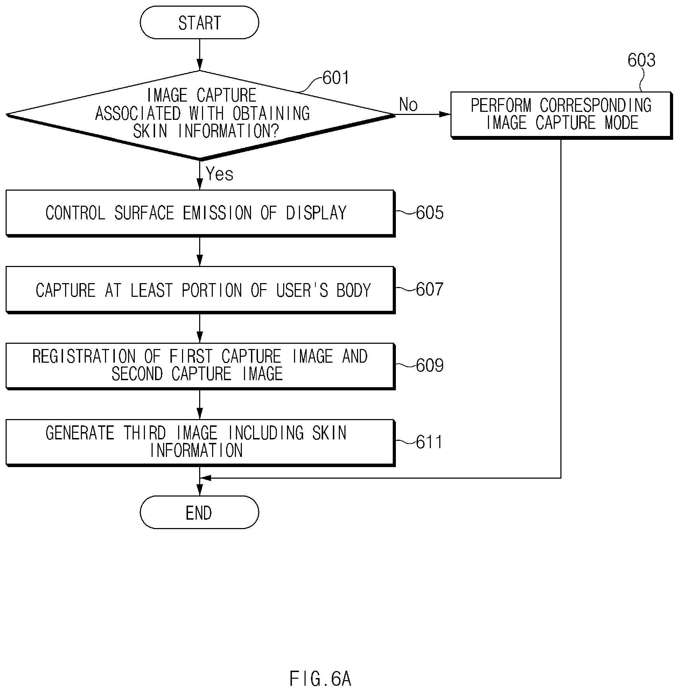

FIG. 6A is a flowchart illustrating a method for providing skin information in an electronic device according to an embodiment of the present disclosure.

Referring to FIG. 6A, in operation 601, a controller (e.g., a controller 140 of FIG. 2) (or a processor) of an electronic device (e.g., an electronic device 100 of FIG. 2) may determine an image capture mode selected by a user input on an execution screen where a specified application (e.g., a camera application) is executed. In this regard, at least one object (e.g., a tap button) associated with setting an image capture mode of an image capture unit (e.g., an image capture unit 120 of FIG. 2) (or an image capture module) of the electronic device may be included on the execution screen where the application is executed. For example, an object associated with activating at least one of a beauty image capture mode, a panorama image capture mode, or a continuous image capture mode may be included on the execution screen.

In an embodiment of the present disclosure, if one of the panorama image capture mode or the continuous image capture mode is activated by a user input, in operation 603, the controller may control to drive at least one of a first camera (e.g., a first camera 125 of FIG. 2) located on a front surface of the electronic device or a third camera (not shown) located on a rear surface of the electronic device to capture a region around the electronic device.

Alternatively, if a beauty image capture mode associated with determining a user skin state through image capture is activated in operation 601, in operation 605, the controller may convert the execution screen where the application is executed into another execution screen or a related user interface for supporting to control surface emission of a display unit (e.g., a display unit 150 of FIG. 2) (or a display) of the electronic device. In an embodiment of the present disclosure, a user may set a light of a specific wavelength band associated with surface emission of the display unit on the other execution screen or the user interface. For example, the user may set the display unit such that the display unit performs surface emission with a light of a first wavelength band (e.g., a light of a red wavelength band, a light of a green wavelength band, or a light of a blue wavelength band) and may set the display unit such that at least two of lights of first to third wavelength bands (e.g., a light of a red wavelength band, a light of a green wavelength band, and a light of a blue wavelength band) have a specified time difference (e.g., a time difference within a range of 0.5 seconds to 1 second) and perform surface emission. If the setting of the surface emission of the display unit (the setting of a light of a specified wavelength band) is completed, the controller may control to start to drive the first camera. Thus, at least a portion of a user's body may be displayed on a screen region of the display unit. In an embodiment of the present disclosure, if a user input for instructing to perform image capture of the first camera is provided to an input interface (e.g., a physical key button) on the electronic device or if a timer set in connection with image capture elapses, the screen region of the display unit may perform surface emission with a light of a set specific wavelength band and may be displayed in a color of the light.

In operation 607, in response to surface emission of the display unit, the controller may control a function operation of at least one of a light source module (e.g., a light source module 121 of FIG. 2), the first camera, or a second camera (e.g., a second camera 127 of FIG. 2), included in the image capture unit. In this case, the light source module may emit, for example, an infrared ray to a front of the electronic device and may receive or detect an infrared ray reflected from one region of a user's body located in front of the electronic device to generate a first image based on an infrared ray on the one region of the user's body. Further, the first camera may receive a light of a specific wavelength band reflected from the one region of the user's body to generate a second image based on surface emission, depending on surface emission of the display unit based on the light of the specific wavelength band. In an embodiment of the present disclosure, if a light of a specific wavelength band due to surface emission of the display unit is set to a plurality of lights, the second image may be generated as a plurality of images in response to a plurality of numbers of surface emission by each light of the specific wavelength band.

In operation 609, the controller may perform registration of the first image and at least one second image through specified image processing. As an example of the registration, the controller may extract a contour for at least one region of a user's body from each of the first image and the at least one second image and may identify at least one feature point on an inner region of the contour. The controller may perform registration of the first image and the at least one second image such that feature points corresponding to each other on the first image and the at least one second image are identical to each other.

In operation 611, the controller may generate a third image through the registration and may perform a series of analysis processes for the third image. The controller may obtain skin information about one region of a user's body corresponding to the third image based on the analyzed result. For example, the controller may compare a color according to each pixel of the third image with a specified skin tone index and may collect skin tone information about each pixel (e.g., average a plurality of skin tone information or extract weighty information among the plurality of skin tone information) to obtain skin tone information about the one region of the user's body. Alternatively, the controller may obtain information about a melanin index or an erythema index for at least one region of the user's body based on the third image. In this regard, the controller may determine a light of a specific wavelength band corresponding to a color of each pixel of the third image with reference to a database constructed in a storage unit (e.g., a storage unit 130 of FIG. 2) (or a memory) and may generate a wave spectrum for the light of the specific wavelength band determined for each pixel. In an embodiment of the present disclosure, the controller may determine a melanin index of a region of a user's body corresponding to a specific pixel based on a gradient of a wave spectrum generated for the specific pixel. For example, the controller may determine that the region of the user's body has a higher melanin index as a gradient of the wave spectrum is sharper. Alternatively, the controller may determine an erythema index of a region of the user's body corresponding to a specific pixel based on an area of a wave spectrum generated for the specific pixel. The controller may determine that a region of the user's body has a higher erythema level as the area is wider. The controller may output the obtained or determined skin information (e.g., skin tone information, melanin index information, or erythema index information) about the one region of the user's body in response to a user input performed on an execution screen where a specified application (e.g., a gallery application or a health management service application) is executed.

A method for providing skin information in an electronic device according to various embodiments may include capturing an image of at least a portion of a user's body based on a light source device configured to emit an electromagnetic wave of a specified wavelength band, a first camera, and a second camera configured to detect the electromagnetic wave, emitting light in a specified color from at least one region of a display of the electronic device based on driving at least one pixel of the display, controlling to drive the image capture device to obtain a first capture image by the first camera and a second capture image by the second camera when the light is emitted light in the specified color, and performing registration of the first capture image and the second capture image to generate a third image.

According to various embodiments of the present disclosure, the method may further include constructing a database for an image color corresponding to a light of a specific wavelength band and determining a light of a specific wavelength band corresponding to a color of each pixel of the third image with reference to the database.

According to various embodiments of the present disclosure, the determining of the light of the specific wavelength band may include generating a spectrum for a light of a specific wavelength band corresponding to a specific pixel of the third image and determining a skin state for one region of a user's body corresponding to the specific pixel based on the spectrum.

According to various embodiments of the present disclosure, the determining of the skin state may include determining a melanin index for the one region of the user's body based on a gradient of the spectrum and determining an erythema index for the one region of the user's body based on an area of the spectrum.

According to various embodiments of the present disclosure, the generating of the third image may include collecting color information about each pixel of the third image and determining a skin tone for one region of a user's body corresponding to the third image.

According to various embodiments of the present disclosure, the method may further include outputting a first user interface including the first capture image or a correction image in which a specified effect is assigned to the first capture image.

According to various embodiments of the present disclosure, the outputting of the first user interface may include, if a user input event on one region of the first capture image or the correction image occurs, identifying a pixel corresponding to a user input region on the first capture image or the correction image among at least one pixel of the third image and outputting skin information about one region of a user's body corresponding to the identified pixel.

According to various embodiments of the present disclosure, the method may further communicating with at least one specified external device.

According to various embodiments of the present disclosure, the communicating may include transmitting the skin information to the at least one specified external device and receiving at least one information corresponding to the skin information from the at least one specified external device.

According to various embodiments of the present disclosure, the method may further include, if an event of activating a specified image capture mode associated with determining a skin state through image capture occurs, outputting a second user interface capable of setting a light of at least one specific wavelength band in connection with light emission of the display.

According to various embodiments of the present disclosure, the outputting of the second user interface may include, if the at least one region of the display emits light with the light of the specified wavelength band, controlling to drive at least one of the light source device, the first camera, and the second camera.

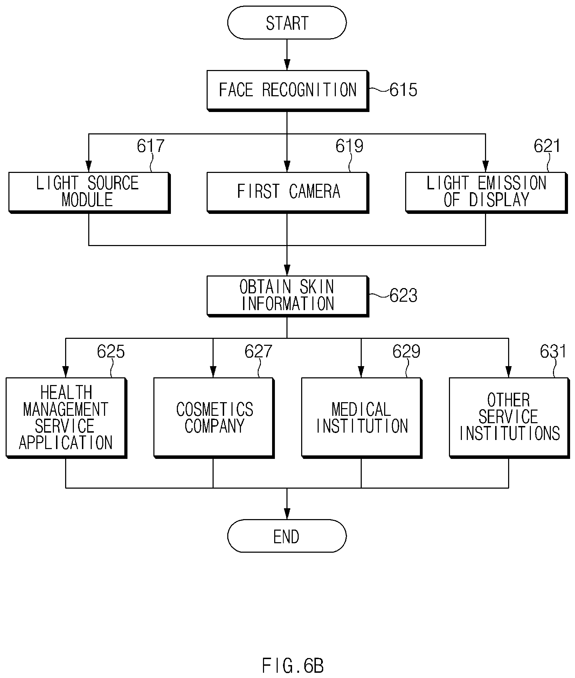

FIG. 6B illustrates a service platform associated with providing skin information in an electronic device according to an embodiment of the present disclosure.

Referring to FIG. 6B, in operation 615, a controller (e.g., a controller 140 of FIG. 2) (or a processor) may recognize one region (e.g., a face) of a user's body located in front of an electronic device (e.g., an electronic device 100 of FIG. 2). For example, if a specified image capture mode associated with driving an image capture unit (e.g., an image capture unit 120 of FIG. 2) (or an image capture module) (e.g., a beauty image capture mode associated with determining a user skin state) is activated and if a setting of a light of a specific wavelength band associated with surface emission of a display unit (e.g., a display unit 150 of FIG. 2) (or a display) is completed, the controller may recognize a user's body. In this regard, the controller may control to drive a first camera (e.g., a first camera 125 of FIG. 2) (e.g., a front camera) to display one region of the user's body on a screen region of the display unit. In this operation, the controller may output an object (e.g., a contour) which functions as a focus guide for a specific region (e.g., a face) of the user's body on the screen region. In an embodiment of the present disclosure, the controller may determine whether the one region of the user's body corresponds to the object. If the one region of the user's body corresponds to the object at a specified threshold rate or more, the controller may recognize the one region of the user's body as the face. Alternatively, the controller may recognize a user face by extracting at least one feature point for the one region of the user's body displayed on the screen region and mapping the extracted feature point with a previously stored feature point on the user face. Alternatively, the controller may recognize the user face by controlling to drive a light source module (e.g., a light source module 121 of FIG. 2) (e.g., an IR LED or an IR LD) and a second camera (e.g., a second camera 127 of FIG. 2) of the image capture unit and identifying an iris of a user.

If the recognition of the at least one region of the user's body is completed, at least one of operation 617, 619, or 621 may be performed. The at least one of operation 617, 619, or 621 may be performed at the same time or in a specified order. For example, after surface emission of the display unit based on a light of a specific wavelength band is performed in operation 621, operations 617 and 619 may be started to generate a first image (e.g., an image based on an infrared ray) and a second image (e.g., an image based on surface emission) by driving of the light source module, the first camera, and the second camera.

If the first image and the second image are generated, in operation 623, the controller may perform a series of processes (e.g., operations 609 and 611 of FIG. 6A) to generate a third image in which registration of the first image and the second image are performed and may obtain skin information about one region of a user's body captured based on the third image.

In operation 625, the controller may store the skin information in one region of a storage unit (e.g., a storage unit 130 of FIG. 2) (or a memory) and may read the stored skin information in response to user control to provide the obtained skin information through a specified application (e.g., a gallery application and/or a health management service application).

Alternatively, in at least one of operation 627, 629, or 631, the controller may transmit the skin information to at least one external device (e.g., a medical institution server, a cosmetics company server, a beauty company server, an online/offline shopping mall company server, or the like) which constructs a network with the electronic device, in response to user control or specified scheduling information. In this operation, the controller may transmit only at least a portion of the skin information to at least one external device depending on user control. In an embodiment of the present disclosure, the user may visit an institution or company which operates the external device or may request the institution or company to remotely provide a questionnaire, or may request the institution or company to recommend a beauty product corresponding to the transmitted skin information, based on the transmission of the skin information to the external device. Thus, the controller may receive feedback information or product information associated with the request, provided from the institution or company, and may output the feedback information or the product information depending on user control.

FIG. 7 illustrates an electronic device in a network environment according to an embodiment of the present disclosure.

Referring to FIG. 7, an electronic device 701 in a network environment 700 according to various embodiments of the present disclosure will be described with reference to FIG. 7. The electronic device 701 may include a bus 710, a processor 720, a memory 730, an input/output interface 750, a display 760, and a communication interface 770. In various embodiments of the present disclosure, at least one of the foregoing elements may be omitted or another element may be added to the electronic device 701.

The bus 710 may include a circuit for connecting the above-mentioned elements 710 to 770 to each other and transferring communications (e.g., control messages and/or data) among the above-mentioned elements.

The processor 720 may include at least one of a CPU, an application processor (AP), or a communication processor (CP). The processor 720 may perform data processing or an operation related to communication and/or control of at least one of the other elements of the electronic device 701.

The memory 730 may include a volatile memory and/or a nonvolatile memory. The memory 730 may store instructions or data related to at least one of the other elements of the electronic device 701. According to an embodiment of the present disclosure, the memory 730 may store software and/or a program 740. The program 740 may include, for example, a kernel 741, a middleware 743, an application programming interface (API) 745, and/or an application program (or an application) 747. At least a portion of the kernel 741, the middleware 743, or the API 745 may be referred to as an operating system (OS).

The kernel 741 may control or manage system resources (e.g., the bus 710, the processor 720, the memory 730, or the like) used to perform operations or functions of other programs (e.g., the middleware 743, the API 745, or the application program 747). Furthermore, the kernel 741 may provide an interface for allowing the middleware 743, the API 745, or the application program 747 to access individual elements of the electronic device 701 in order to control or manage the system resources.

The middleware 743 may serve as an intermediary so that the API 745 or the application program 747 communicates and exchanges data with the kernel 741.

Furthermore, the middleware 743 may handle one or more task requests received from the application program 747 according to a priority order. For example, the middleware 743 may assign at least one application program 747 a priority for using the system resources (e.g., the bus 710, the processor 720, the memory 730, or the like) of the electronic device 701. For example, the middleware 743 may handle the one or more task requests according to the priority assigned to the at least one application, thereby performing scheduling or load balancing with respect to the one or more task requests.

The API 745, which is an interface for allowing the application program 747 to control a function provided by the kernel 741 or the middleware 743, may include, for example, at least one interface or function (e.g., instructions) for file control, window control, image processing, character control, or the like.

The input/output interface 750 may serve to transfer an instruction or data input from a user or another external device to (an)other element(s) of the electronic device 701. Furthermore, the input/output interface 750 may output instructions or data received from (an)other element(s) of the electronic device 701 to the user or another external device.

The display 760 may include, for example, a liquid crystal display (LCD), a light-emitting diode (LED) display, an organic light-emitting diode (OLED) display, a microelectromechanical systems (MEMS) display, or an electronic paper display. The display 760 may present various content (e.g., a text, an image, a video, an icon, a symbol, or the like) to the user. The display 760 may include a touch screen, and may receive a touch, gesture, proximity or hovering input from an electronic pen or a part of a body of the user.

The communication interface 770 may set communications between the electronic device 701 and an external device (e.g., a first external electronic device 702, a second external electronic device 704, or a server 706). For example, the communication interface 770 may be connected to a network 762 via wireless communications or wired communications so as to communicate with the external device (e.g., the second external electronic device 704 or the server 706).

The wireless communications may employ at least one of cellular communication protocols, such as long-term evolution (LTE), LTE-advance (LTE-A), code division multiple access (CDMA), wideband CDMA (WCDMA), universal mobile telecommunications system (UMTS), wireless broadband (WiBro), or global system for mobile communications (GSM). The wireless communications may include, for example, a short-range communications 764. The short-range communications may include at least one of wireless fidelity (Wi-Fi), Bluetooth, near field communication (NFC), magnetic stripe transmission (MST), or GNSS.

The MST may generate pulses according to transmission data and the pulses may generate electromagnetic signals. The electronic device 701 may transmit the electromagnetic signals to a reader device, such as a POS (point of sales) device. The POS device may detect the magnetic signals by using a MST reader and restore data by converting the detected electromagnetic signals into electrical signals.

The GNSS may include, for example, at least one of global positioning system (GPS), global navigation satellite system (GLONASS), BeiDou navigation satellite system (BeiDou), or Galileo, the European global satellite-based navigation system according to a use area or a bandwidth. Hereinafter, the term "GPS" and the term "GNSS" may be interchangeably used. The wired communications may include at least one of universal serial bus (USB), high definition multimedia interface (HDMI), recommended standard 232 (RS-232), plain old telephone service (POTS), or the like. The network 762 may include at least one of telecommunications networks, for example, a computer network (e.g., local area network (LAN) or wide area network (WAN)), the Internet, or a telephone network.

The types of the first external electronic device 702 and the second external electronic device 704 may be the same as or different from the type of the electronic device 701. According to an embodiment of the present disclosure, the server 706 may include a group of one or more servers. A portion or all of operations performed in the electronic device 701 may be performed in one or more other electronic devices (e.g., the first external electronic device 702, the second external electronic device 704, or the server 706). When the electronic device 701 should perform a certain function or service automatically or in response to a request, the electronic device 701 may request at least a portion of functions related to the function or service from another device (e.g., the first external electronic device 702, the second external electronic device 704, or the server 706) instead of or in addition to performing the function or service for itself. The other electronic device (e.g., the first external electronic device 702, the second external electronic device 704, or the server 706) may perform the requested function or additional function, and may transfer a result of the performance to the electronic device 701. The electronic device 701 may use a received result itself or additionally process the received result to provide the requested function or service. To this end, for example, a cloud computing technology, a distributed computing technology, or a client-server computing technology may be used.

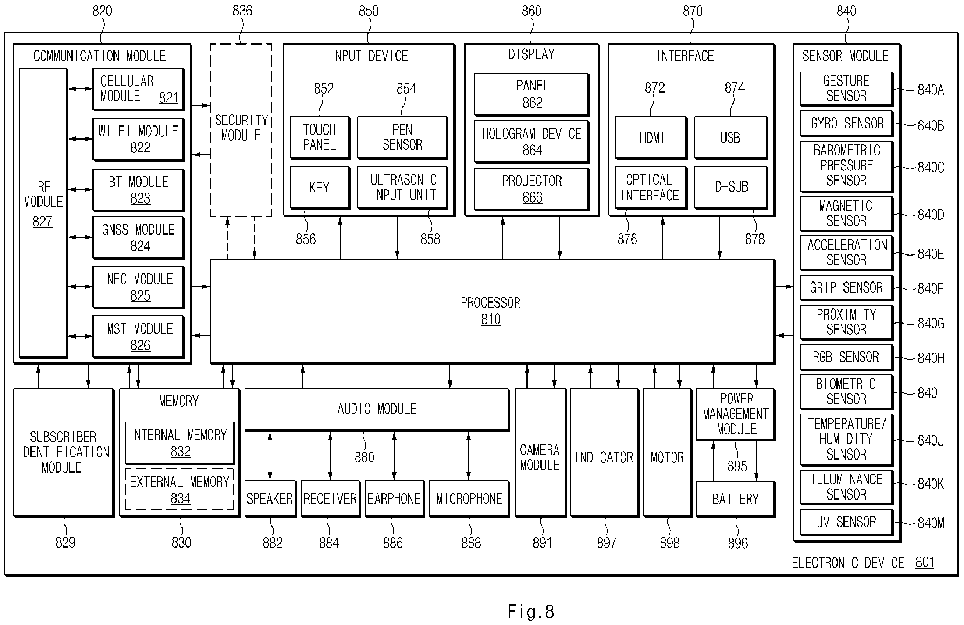

FIG. 8 is a block diagram illustrating an electronic device according to an embodiment of the present disclosure.

Referring to FIG. 8, an electronic device 801 may include, for example, all or part of an electronic device 701 shown in FIG. 7. The electronic device 801 may include one or more processors 810 (e.g., application processors (APs)), a communication module 820, a subscriber identification module (SIM) 829, a memory 830, a security module 836, a sensor module 840, an input device 850, a display 860, an interface 870, an audio module 880, a camera module 891, a power management module 895, a battery 896, an indicator 897, and a motor 898.

The processor 810 may drive, for example, an operating system (OS) or an application program to control a plurality of hardware or software components connected thereto and may process and compute a variety of data. The processor 810 may be implemented with, for example, a system on chip (SoC). According to an embodiment of the present disclosure, the processor 810 may include a graphics processing unit (GPU) (not shown) and/or an image signal processor (not shown). The processor 810 may include at least some (e.g., a cellular module 821) of the components shown in FIG. 8. The processor 810 may load a command or data received from at least one of other components (e.g., a non-volatile memory) into a volatile memory to process the data and may store various data in a non-volatile memory.

The communication module 820 may have the same or similar configuration to the communication interface 770 of FIG. 7. The communication module 820 may include, for example, the cellular module 821, a Wi-Fi module 822, a Bluetooth (BT) module 823, a global navigation satellite system (GNSS) module 824 (e.g., a GPS module, a Glonass module, a Beidou module, or a Galileo module), a near field communication (NFC) module 825, an MST module 826, and a radio frequency (RF) module 827.

The cellular module 821 may provide, for example, a voice call service, a video call service, a text message service, or an Internet service, and the like through a communication network. According to an embodiment of the present disclosure, the cellular module 821 may identify and authenticate the electronic device 801 in a communication network using the SIM 829 (e.g., a SIM card). According to an embodiment of the present disclosure, the cellular module 821 may perform at least part of functions which may be provided by the processor 810. According to an embodiment of the present disclosure, the cellular module 821 may include a CP.

The Wi-Fi module 822, the BT module 823, the GNSS module 824, the NFC module 825, or the MST module 826 may include, for example, a processor for processing data transmitted and received through the corresponding module. According to various embodiments of the present disclosure, at least some (e.g., two or more) of the cellular module 821, the Wi-Fi module 822, the BT module 823, the GNSS module 824, the NFC module 825, or the MST module 826 may be included in one integrated chip (IC) or one IC package.

The RF module 827 may transmit and receive, for example, a communication signal (e.g., an RF signal). Though not shown, the RF module 827 may include, for example, a transceiver, a power amplifier module (PAM), a frequency filter, or a low noise amplifier (LNA), or an antenna, and the like. According to another embodiment of the present disclosure, at least one of the cellular module 821, the Wi-Fi module 822, the BT module 823, the GNSS module 824, the NFC module 825, or the MST module 826 may transmit and receive an RF signal through a separate RF module.

The SIM 829 may include, for example, a card which includes a SIM and/or an embedded SIM. The SIM 829 may include unique identification information (e.g., an integrated circuit card identifier (ICCID)) or subscriber information (e.g., an international mobile subscriber identity (IMSI)).

The memory 830 (e.g., a memory 730 of FIG. 7) may include, for example, an embedded memory 832 or an external memory 834. The embedded memory 832 may include at least one of, for example, a volatile memory (e.g., a dynamic random access memory (DRAM), a static RAM (SRAM), a synchronous dynamic RAM (SDRAM), and the like), or a non-volatile memory (e.g., a one-time programmable read only memory (OTPROM), a programmable ROM (PROM), an erasable and programmable ROM (EPROM), an electrically erasable and programmable ROM (EEPROM), a mask ROM, a flash ROM, a flash memory (e.g., a NAND flash memory or a NOR flash memory, and the like), a hard drive, or a solid state drive (SSD)).

The external memory 834 may include a flash drive, for example, a compact flash (CF), a secure digital (SD), a micro-SD, a mini-SD, an extreme digital (xD), a multimedia card (MMC), or a memory stick, and the like. The external memory 834 may operatively and/or physically connect with the electronic device 801 through various interfaces.

The security module 836 may be a module which has a relatively higher secure level than the memory 830 and may be a circuit which stores secure data and guarantees a protected execution environment. The security module 836 may be implemented with a separate circuit and may include a separate processor. The security module 836 may include, for example, an embedded secure element (eSE) which is present in a removable smart chip or a removable SD card or is embedded in a fixed chip of the electronic device 801. In addition, the security module 836 may be driven by an OS different from the OS of the electronic device 801. For example, the security module 836 may operate based on a java card open platform (JCOP) OS.

The sensor module 840 may measure, for example, a physical quantity or may detect an operation state of the electronic device 801, and may convert the measured or detected information to an electric signal. The sensor module 840 may include at least one of, for example, a gesture sensor 840A, a gyro sensor 840B, a barometric pressure sensor 840C, a magnetic sensor 840D, an acceleration sensor 840E, a grip sensor 840F, a proximity sensor 840G, a color sensor 840H (e.g., red, green, blue (RGB) sensor), a biometric sensor 8401, a temperature/humidity sensor 840J, an illumination sensor 840K, or an ultraviolet (UV) sensor 840M. Additionally or alternatively, the sensor module 840 may further include, for example, an e-nose sensor (not shown), an electromyography (EMG) sensor (not shown), an electroencephalogram (EEG) sensor (not shown), an electrocardiogram (ECG) sensor (not shown), an IR sensor (not shown), an iris sensor (not shown), and/or a fingerprint sensor (not shown), and the like. The sensor module 840 may further include a control circuit for controlling at least one or more sensors included therein. According to various embodiments of the present disclosure, the electronic device 801 may further include a processor configured to control the sensor module 840, as part of the processor 810 or to be independent of the processor 810. While the processor 810 is in a sleep state, the electronic device 801 may control the sensor module 840.

The input device 850 may include, for example, a touch panel 852, a (digital) pen sensor 854, a key 856, or an ultrasonic input device 858. The touch panel 852 may use at least one of, for example, a capacitive type, a resistive type, an infrared type, or an ultrasonic type. In addition, the touch panel 852 may further include a control circuit. The touch panel 852 may further include a tactile layer and may provide a tactile reaction to a user.

The (digital) pen sensor 854 may be, for example, part of the touch panel 852 or may include a separate sheet for recognition. The key 856 may include, for example, a physical button, an optical key, or a keypad. The ultrasonic input device 858 may allow the electronic device 801 to detect a sound wave using a microphone (e.g., a microphone 888) and to verify data through an input tool generating an ultrasonic signal.

The display 860 (e.g., a display 760 of FIG. 7) may include a panel 862, a hologram device 864, or a projector 866. The panel 862 may include the same or similar configuration to the display 760. The panel 862 may be implemented to be, for example, flexible, transparent, or wearable. The panel 862 and the touch panel 852 may be integrated into one module. The hologram device 864 may show a stereoscopic image in a space using interference of light. The projector 866 may project light onto a screen to display an image. The screen may be positioned, for example, inside or outside the electronic device 801. According to an embodiment of the present disclosure, the display 860 may further include a control circuit for controlling the panel 862, the hologram device 864, or the projector 866.

The interface 870 may include, for example, an HDMI 872, a USB 874, an optical interface 876, or a D-subminiature 878. The interface 870 may be included in, for example, the communication interface 770 shown in FIG. 7. Additionally or alternatively, the interface 870 may include, for example, a mobile high definition link (MHL) interface, an SD card/MMC interface, or an infrared data association (IrDA) standard interface.

The audio module 880 may convert a sound and an electric signal in dual directions. At least part of components of the audio module 880 may be included in, for example, an input and output interface 750 (or a user interface) shown in FIG. 7. The audio module 880 may process sound information input or output through, for example, a speaker 882, a receiver 884, an earphone 886, or the microphone 888, and the like.

The camera module 891 may be a device which captures a still image and a moving image. According to an embodiment of the present disclosure, the camera module 891 may include one or more image sensors (not shown) (e.g., a front sensor or a rear sensor), a lens (not shown), an image signal processor (ISP) (not shown), or a flash (not shown) (e.g., an LED or a xenon lamp).

The power management module 895 may manage, for example, power of the electronic device 801. According to an embodiment of the present disclosure, though not shown, the power management module 895 may include a power management integrated circuit (PMIC), a charger IC or a battery or fuel gauge. The PMIC may have a wired charging method and/or a wireless charging method. The wireless charging method may include, for example, a magnetic resonance method, a magnetic induction method, or an electromagnetic method, and the like. An additional circuit for wireless charging, for example, a coil loop, a resonance circuit, or a rectifier, and the like may be further provided. The battery gauge may measure, for example, the remaining capacity of the battery 896 and voltage, current, or temperature thereof while the battery 896 is charged. The battery 896 may include, for example, a rechargeable battery or a solar battery.

The indicator 897 may display a specific state of the electronic device 801 or part (e.g., the processor 810) thereof, for example, a booting state, a message state, or a charging state, and the like. The motor 898 may convert an electric signal into mechanical vibration and may generate vibration or a haptic effect, and the like. Though not shown, the electronic device 801 may include a processing unit (e.g., a GPU) for supporting a mobile TV. The processing unit for supporting the mobile TV may process media data according to standards, for example, a digital multimedia broadcasting (DMB) standard, a digital video broadcasting (DVB) standard, or a MediaFLO™ standard, and the like.

Each of the above-mentioned elements of the electronic device according to various embodiments of the present disclosure may be configured with one or more components, and names of the corresponding elements may be changed according to the type of the electronic device. The electronic device according to various embodiments of the present disclosure may include at least one of the above-mentioned elements, some elements may be omitted from the electronic device, or other additional elements may be further included in the electronic device. In addition, some of the elements of the electronic device according to various embodiments of the present disclosure may be combined with each other to form one entity, thereby making it possible to perform the functions of the corresponding elements in the same manner as before the combination.

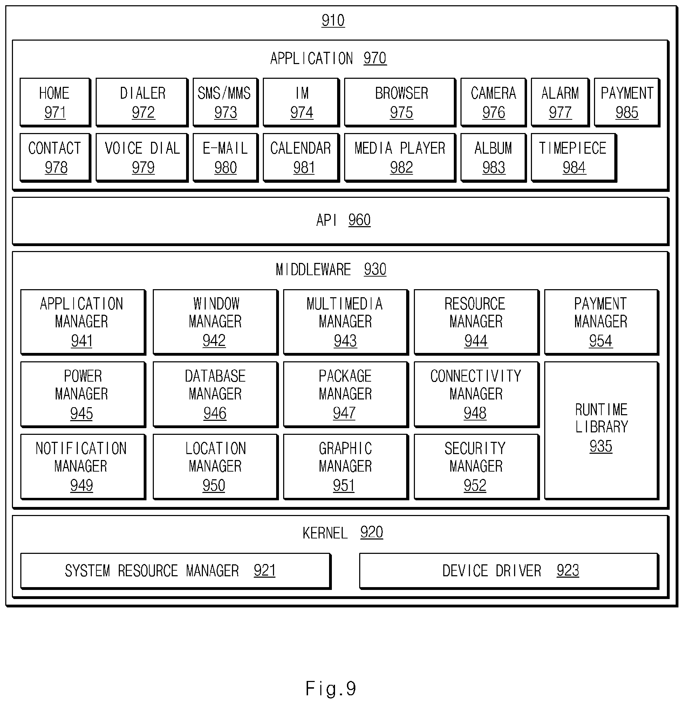

FIG. 9 is a block diagram illustrating a program module according to an embodiment of the present disclosure.

Referring to FIG. 9, according to an embodiment of the present disclosure, a program module 910 (e.g., a program 740 of FIG. 7) may include an operating system (OS) for controlling resources associated with an electronic device (e.g., an electronic device 701 of FIG. 7) and/or various applications (e.g., an application program 747 of FIG. 7) which are executed on the OS. The OS may be, for example, Android, iOS, Windows, Symbian, Tizen, or Bada, and the like.

The program module 910 may include a kernel 920, a middleware 930, an API 960, and/or an application 970. At least part of the program module 910 may be preloaded on the electronic device, or may be downloaded from an external electronic device (e.g., a first external electronic device 702, a second external electronic device 704, or a server 706, and the like of FIG. 7).

The kernel 920 (e.g., a kernel 741 of FIG. 7) may include, for example, a system resource manager 921 and/or a device driver 923. The system resource manager 921 may control, assign, or collect, and the like system resources. According to an embodiment of the present disclosure, the system resource manager 921 may include a process management unit, a memory management unit, or a file system management unit, and the like. The device driver 923 may include, for example, a display driver, a camera driver, a BT driver, a shared memory driver, a USB driver, a keypad driver, a Wi-Fi driver, an audio driver, or an inter-process communication (IPC) driver.

The middleware 930 (e.g., a middleware 743 of FIG. 7) may provide, for example, functions the application 970 needs in common, and may provide various functions to the application 970 through the API 960 such that the application 970 efficiently uses limited system resources in the electronic device. According to an embodiment of the present disclosure, the middleware 930 (e.g., the middleware 743) may include at least one of a runtime library 935, an application manager 941, a window manager 942, a multimedia manager 943, a resource manager 944, a power manager 945, a database manager 946, a package manager 947, a connectivity manager 948, a notification manager 949, a location manager 950, a graphic manager 951, a security manager 952, or a payment manager 954.

The runtime library 935 may include, for example, a library module used by a compiler to add a new function through a programming language while the application 970 is executed. The runtime library 935 may perform a function about input and output management, memory management, or an arithmetic function.

The application manager 941 may manage, for example, a life cycle of at least one of the application 970. The window manager 942 may manage graphic user interface (GUI) resources used on a screen of the electronic device. The multimedia manager 943 may determine a format utilized for reproducing various media files and may encode or decode a media file using a codec corresponding to the corresponding format. The resource manager 944 may manage source codes of at least one of the application 970, and may manage resources of a memory or a storage space, and the like.

The power manager 945 may act together with, for example, a basic input/output system (BIOS) and the like, may manage a battery or a power source, and may provide power information utilized for an operation of the electronic device. The database manager 946 may generate, search, or change a database to be used in at least one of the application 970. The package manager 947 may manage installation or update of an application distributed by a type of a package file.

The connectivity manager 948 may manage, for example, wireless connection, such as Wi-Fi connection or BT connection, and the like. The notification manager 949 may display or notify events, such as an arrival message, an appointment, and proximity notification, by a method which is not disturbed to the user. The location manager 950 may manage location information of the electronic device. The graphic manager 951 may manage a graphic effect to be provided to the user or a user interface (UI) related to the graphic effect. The security manager 952 may provide all security functions utilized for system security or user authentication, and the like. According to an embodiment of the present disclosure, when the electronic device (e.g., an electronic device 701 of FIG. 7) has a phone function, the middleware 930 may further include a telephony manager (not shown) for managing a voice or video communication function of the electronic device.

The middleware 930 may include a middleware module which configures combinations of various functions of the above-described components. The middleware 930 may provide a module which specializes according to kinds of OSs to provide a differentiated function. In addition, the middleware 930 may dynamically delete some of old components or may add new components.

The API 960 (e.g., an API 745 of FIG. 7) may be, for example, a set of API programming functions, and may be provided with different components according to OSs. For example, in case of Android or iOS, one API set may be provided according to platforms. In case of Tizen, two or more API sets may be provided according to platforms.

The application 970 (e.g., an application program 747 of FIG. 7) may include one or more of, for example, a home application 971, a dialer application 972, a short message service/multimedia message service (SMS/MMS) application 973, an instant message (IM) application 974, a browser application 975, a camera application 976, an alarm application 977, a contact application 978, a voice dial application 979, an e-mail application 980, a calendar application 981, a media player application 982, an album application 983, a clock application 984, a payment application 985, a health care application (e.g., an application for measuring quantity of exercise or blood sugar, and the like), or an environment information application (e.g., an application for providing atmospheric pressure information, humidity information, or temperature information, and the like), and the like.

According to an embodiment of the present disclosure, the application 970 may include an application (hereinafter, for better understanding and ease of description, referred to as "information exchange application") for exchanging information between the electronic device (e.g., the electronic device 701 of FIG. 7) and an external electronic device (e.g., the first external electronic device 702 or the second external electronic device 704). The information exchange application may include, for example, a notification relay application for transmitting specific information to the external electronic device or a device management application for managing the external electronic device.

For example, the notification relay application may include a function of transmitting notification information, which is generated by other applications (e.g., the SMS/MMS application, the e-mail application, the health care application, or the environment information application, and the like) of the electronic device, to the external electronic device (e.g., the first external electronic device 702 or the second external electronic device 704). In addition, the notification relay application may receive, for example, notification information from the external electronic device, and may provide the received notification information to the user of the electronic device.

The device management application may manage (e.g., install, delete, or update), for example, at least one (e.g., a function of turning on/off the external electronic device itself (or partial components) or a function of adjusting brightness (or resolution) of a display) of functions of the external electronic device (e.g., the first external electronic device 702 or the second external electronic device 704) which communicates with the electronic device, an application which operates in the external electronic device, or a service (e.g., a call service or a message service) provided from the external electronic device.

According to an embodiment of the present disclosure, the application 970 may include an application (e.g., the health card application of a mobile medical device) which is preset according to attributes of the external electronic device (e.g., the first external electronic device 702 or the second external electronic device 704). According to an embodiment of the present disclosure, the application 970 may include an application received from the external electronic device (e.g., the server 706, the first external electronic device 702, or the second external electronic device 704). According to an embodiment of the present disclosure, the application 970 may include a preloaded application or a third party application which may be downloaded from a server. Names of the components of the program module 910 according to various embodiments of the present disclosure may differ according to kinds of OSs.

According to various embodiments of the present disclosure, at least part of the program module 910 may be implemented with software, firmware, hardware, or at least two or more combinations thereof. At least part of the program module 910 may be implemented (e.g., executed) by, for example, a processor (e.g., a processor 810). At least part of the program module 910 may include, for example, a module, a program, a routine, sets of instructions, or a process, and the like for performing one or more functions.

The term "module" used herein may represent, for example, a unit including one of hardware, software and firmware or a combination thereof. The term "module" may be interchangeably used with the terms "unit", "logic", "logical block", "component" and "circuit". The "module" may be a minimum unit of an integrated component or may be a part thereof. The "module" may be a minimum unit for performing one or more functions or a part thereof. The "module" may be implemented mechanically or electronically. For example, the "module" may include at least one of an application-specific integrated circuit (ASIC) chip, a field-programmable gate array (FPGA), and a programmable-logic device for performing some operations, which are known or will be developed.

At least a part of devices (e.g., modules or functions thereof) or methods (e.g., operations) according to various embodiments of the present disclosure may be implemented as instructions stored in a computer-readable storage medium in the form of a program module. In the case where the instructions are performed by a processor (e.g., the processor 720), the processor may perform functions corresponding to the instructions. The computer-readable storage medium may be, for example, the memory 730.

Certain aspects of the present disclosure can also be embodied as computer readable code on a non-transitory computer readable recording medium. A non-transitory computer readable recording medium is any data storage device that can store data which can be thereafter read by a computer system. Examples of the non-transitory computer readable recording medium include a Read-Only Memory (ROM), a Random-Access Memory (RAM), Compact Disc-ROMs (CD-ROMs), magnetic tapes, floppy disks, and optical data storage devices. The non-transitory computer readable recording medium can also be distributed over network coupled computer systems so that the computer readable code is stored and executed in a distributed fashion. In addition, functional programs, code, and code segments for accomplishing the present disclosure can be easily construed by programmers skilled in the art to which the present disclosure pertains.

At this point it should be noted that the various embodiments of the present disclosure as described above typically involve the processing of input data and the generation of output data to some extent. This input data processing and output data generation may be implemented in hardware or software in combination with hardware. For example, specific electronic components may be employed in a mobile device or similar or related circuitry for implementing the functions associated with the various embodiments of the present disclosure as described above. Alternatively, one or more processors operating in accordance with stored instructions may implement the functions associated with the various embodiments of the present disclosure as described above. If such is the case, it is within the scope of the present disclosure that such instructions may be stored on one or more non-transitory processor readable mediums. Examples of the processor readable mediums include a ROM, a RAM, CD-ROMs, magnetic tapes, floppy disks, and optical data storage devices. The processor readable mediums can also be distributed over network coupled computer systems so that the instructions are stored and executed in a distributed fashion. In addition, functional computer programs, instructions, and instruction segments for accomplishing the present disclosure can be easily construed by programmers skilled in the art to which the present disclosure pertains.

A module or a program module according to various embodiments of the present disclosure may include at least one of the above-mentioned elements, or some elements may be omitted or other additional elements may be added. Operations performed by the module, the program module or other elements according to various embodiments of the present disclosure may be performed in a sequential, parallel, iterative or heuristic way. Furthermore, some operations may be performed in another order or may be omitted, or other operations may be added.

While the present disclosure has been shown and described with reference to various embodiments thereof, it will be understood by those skilled in the art that various changes in form and details may be made therein without departing from the scope of the present disclosure as defined by the appended claims.

## Claims

1. An electronic device (100) comprising:
an image capture device (120) comprising a light source device (121) configured to emit an infrared ray, a first camera (125), and a second camera (127) configured to detect the infrared ray and to capture an image of at least a portion of a user's body based on the emitted infrared ray;
a memory (130) configured to store an image captured by the image capture device;
a display (150) configured to emit light in a specified color from at least one region based on driving at least one pixel; and
at least one processor (140) electrically connected with the image capture device, the memory, and the display, the at least one processor configured to:
control to drive the display to emit the light in the specified color from the at least one region,
control to drive the image capture device to obtain a first capture image by the first camera when the display emits the light in the specified color and a second capture image by the second camera when the light source device emits the infrared ray,
generate a third image by performing registration of the first capture image and the second capture image,
generate a spectrum for a light of a specific wavelength band corresponding to a specific pixel of the generated third image, and
determine a skin state for one region of a user's body corresponding to the specific pixel based on a gradient and an area size of the generated spectrum.

2. The electronic device of claim 1, wherein the at least one processor is further configured to:
construct a database for an image color corresponding to a light of a specific wavelength band, and
determine a light of a specific wavelength band corresponding to a color of each pixel of the third image with reference to the database.

3. The electronic device of claim 2, wherein the at least one processor is further configured to:
determine a melanin index for the one region of the user's body based on the gradient of the spectrum, and
determine an erythema index for the one region of the user's body based on the area size of the spectrum.

4. The electronic device of claim 1, wherein the at least one processor is further configured to:
collect color information about each pixel of the third image, and
determine a skin tone for the one region of the user's body corresponding to the third image.

5. The electronic device of claim 1, wherein the at least one processor is further configured to:
output a first user interface including the first capture image or a correction image in which a specified effect is assigned to the first capture image.

6. The electronic device of claim 5, wherein the at least one processor is further configured to:
if a user input event on one region of the first capture image or the correction image occurs, identify a pixel corresponding to a user input region on the first capture image or the correction image among at least one pixel of the third image, and
output skin information about one region of a user's body corresponding to the identified pixel.

7. The electronic device of claim 1, wherein the at least one processor is further configured to:
if an event of activating a specified image capture mode associated with determining a skin state through image capture occurs, output a second user interface capable of setting a light of at least one specific wavelength band in connection with light emission of the display.

8. A method for providing skin information in an electronic device, the method comprising:
capturing (601) an image of at least a portion of a user's body based on a light source device configured to emit an infrared ray, a first camera, and a second camera configured to detect the infrared ray;
emitting (605) light in a specified color from at least one region of a display of the electronic device based on driving at least one pixel of the display;
controlling (607) to drive the image capture device to obtain a first capture image by the first camera when the display emits the light in the specified color and a second capture image by the second camera when the light source device emits the infrared ray;
generating (611) a third image by performing (609) registration of the first capture image and the second capture image,
generating a spectrum for a light of a specific wavelength band corresponding to a specific pixel of the generated third image, and
determining a skin state for one region of a user's body corresponding to the specific pixel based on a gradient and an area size of the generated spectrum.

9. The method of claim 8, further comprising:
constructing a database for an image color corresponding to a light of a specific wavelength band; and
determining a light of a specific wavelength band corresponding to a color of each pixel of the third image with reference to the database.

10. The method of claim 9, wherein the determining of the skin state comprises:
determining a melanin index for the one region of the user's body based on the gradient of the spectrum, and
determining an erythema index for the one region of the user's body based on the area size of the spectrum.

11. The method of claim 8, wherein the generating of the third image comprises:
collecting color information about each pixel of the third image, and
determining a skin tone for the one region of the user's body corresponding to the third image.

12. The method of claim 8, further comprising:
outputting a first user interface including the first capture image or a correction image in which a specified effect is assigned to the first capture image.

13. The method of claim 12, wherein the outputting of the first user interface comprises:
if a user input event on one region of the first capture image or the correction image occurs, identifying a pixel corresponding to a user input region on the first capture image or the correction image among at least one pixel of the third image, and
outputting skin information about one region of a user's body corresponding to the identified pixel.

## Patentansprüche

1. Elektronische Vorrichtung (100), umfassend:
eine Bildaufnahmevorrichtung (120), umfassend eine Lichtquellenvorrichtung (121), die dazu konfiguriert ist, einen Infrarotstrahl auszugeben, eine erste Kamera (125) und eine zweite Kamera (127), die dazu konfiguriert sind, den Infrarotstrahl zu erfassen und ein Bild von mindestens einem Abschnitt des Körpers eines Benutzers auf Grundlage des ausgegebenen Infrarotstrahls aufzunehmen;
einen Speicher (130), der dazu konfiguriert ist, ein Bild zu speichern, das von der Bildaufnahmevorrichtung aufgenommen wurde;
eine Anzeige (150), die dazu konfiguriert ist, Licht in einer bestimmten Farbe von mindestens einem Bereich auf Grundlage der Ansteuerung mindestens eines Pixels auszugeben; und
mindestens einen Prozessor (140), der elektrisch mit der Bildaufnahmevorrichtung, dem Speicher und der Anzeige verbunden ist, wobei der mindestens eine Prozessor zu Folgendem konfiguriert ist:
Steuern, um die Anzeige zum Ausgeben des Lichts in der bestimmten Farbe von dem mindestens einen Bereich anzusteuern,
Steuern, um die Bildaufnahmevorrichtung zu Folgendem anzusteuern: Aufnehmen eines ersten Aufnahmebilds durch die erste Kamera, wenn die Anzeige das Licht in der bestimmten Farbe ausgibt und eines zweiten Aufnahmebilds durch die zweite Kamera, wenn die Lichtquellenvorrichtung den Infrarotstrahl ausgibt, Erzeugen eines dritten Bilds durch Durchführen einer Registrierung des ersten Aufnahmebilds und des zweiten Aufnahmebilds, Erzeugen eines Spektrums für ein Licht eines bestimmten Wellenlängenbands, das einem bestimmten Pixel des erzeugen dritten Bilds entspricht, und Bestimmen eines Hautzustands für einen Bereich des Körpers eines Benutzers, der dem bestimmten Pixel entspricht, auf Grundlage eines Gradienten und einer Flächengröße des erzeugten Spektrums.

2. Elektronische Vorrichtung nach Anspruch 1, wobei der mindestens eine Prozessor ferner zu Folgendem konfiguriert ist:
Konstruieren einer Datenbank für eine Bildfarbe, die einem Licht eines bestimmten Wellenlängenbads entspricht, und Bestimmen eines Lichts eines bestimmten Wellenlängenbands, das einer Farbe jedes Pixels des dritten Bilds entspricht, in Bezug auf die Datenbank.

3. Elektronische Vorrichtung nach Anspruch 2, wobei der mindestens eine Prozessor ferner zu Folgendem konfiguriert ist:
Bestimmen eines Melaninindex für den einen Bereich des Körpers des Benutzers auf Grundlage des Gradienten des Spektrums und Bestimmen eines Erythemindex für den einen Bereich des Körpers des Benutzers auf Grundlage der Flächengröße des Spektrums.

4. Elektronische Vorrichtung nach Anspruch 1, wobei der mindestens eine Prozessor ferner zu Folgendem konfiguriert ist:
Erfassen von Farbinformationen zu jedem Pixel des dritten Bilds und Bestimmen eines Hauttons für den einen Bereich des Körpers des Benutzers, der dem dritten Bild entspricht.

5. Elektronische Vorrichtung nach Anspruch 1, wobei der mindestens eine Prozessor ferner zu Folgendem konfiguriert ist:
Ausgeben einer ersten Benutzerschnittstelle, die das erste Aufnahmebild oder ein Korrekturbild beinhaltet, in dem ein bestimmter Effekt dem ersten Aufnahmebild zugewiesen ist.

6. Elektronische Vorrichtung nach Anspruch 5, wobei der mindestens eine Prozessor ferner zu Folgendem konfiguriert ist:
wenn ein Benutzereingabeereignis auf einem Bereich des ersten Aufnahmebilds oder des Korrekturbilds erfolgt, Identifizieren eines Pixels, das einem Benutzereingabebereich auf dem ersten Aufnahmebild oder dem Korrekturbild entspricht, unter dem mindestens einen Pixel des dritten Bilds und Ausgeben von Hautinformationen zu einem Bereich des Körpers eines Benutzers, der dem identifizierten Pixel entspricht.

7. Elektronische Vorrichtung nach Anspruch 1, wobei der mindestens eine Prozessor ferner zu Folgendem konfiguriert ist:
wenn ein Ereignis des Aktivierens eines bestimmten Bildaufnahmemodus, der dem Bestimmen eines Hautzustands zugeordnet ist, während der Bildaufnahme erfolgt, Ausgeben einer zweiten Benutzerschnittstelle, die zum Einstellen eines Lichts mindestens eines bestimmten Wellenlängenbands in Verbindung mit Lichtemission auf der Anzeige fähig ist.

8. Verfahren zum Bereitstellen von Hautinformationen in einer elektronischen Vorrichtung, das Verfahren umfassend:
Aufnehmen (601) eines Bilds von mindestens einem Abschnitt des Körpers eines Benutzers auf Grundlage einer Lichtquellenvorrichtung, die dazu konfiguriert ist, einen Infrarotstrahl auszugeben, eine erste Kamera und eine zweite Kamera, die dazu konfiguriert sind, den Infrarotstrahl zu erfassen;
Ausgeben (605) eines Lichts in einer bestimmten Farbe von mindestens einem Bereich einer Anzeige der elektronischen Vorrichtung auf Grundlage der Ansteuerung mindestens eines Pixels der Anzeige;
Steuern (607), um die Bilderfassungsvorrichtung zu Folgendem anzusteuern: Erhalten eines ersten Aufnahmebilds durch die erste Kamera, wenn die Anzeige ein Licht in der angegebenen Farbe ausgibt und eines zweiten Aufnahmebilds durch die zweite Kamera, wenn die Lichtquellenvorrichtung den Infrarotstrahl ausgibt;
Erzeugen (611) eines dritten Bilds durch Durchführen (609) einer Registrierung des ersten Aufnahmebilds und des zweiten Aufnahmebilds, Erzeugen eines Spektrums für ein Licht eines bestimmten Wellenlängenbands, das einem bestimmten Pixel des erzeugen dritten Bilds entspricht, und Bestimmen eines Hautzustands für einen Bereich des Körpers eines Benutzers, der dem bestimmten Pixel entspricht, auf Grundlage eines Gradienten und einer Flächengröße des erzeugten Spektrums.

9. Verfahren nach Anspruch 8, ferner umfassend:
Konstruieren einer Datenbank für eine Bildfarbe, die einem Licht eines bestimmten Wellenlängenbands entspricht; und
Bestimmen eines Lichts eines bestimmten Wellenlängenbands, das einer Farbe jedes Pixels des dritten Bilds entspricht, in Bezug auf die Datenbank.

10. Verfahren nach Anspruch 9, wobei das Bestimmen des ersten Hautzustands Folgendes umfasst:
Bestimmen eines Melaninindex für den einen Bereich des Körpers des Benutzers auf Grundlage des Gradienten des Spektrums und Bestimmen eines Erythemindex für den einen Bereich des Körpers des Benutzers auf Grundlage der Flächengröße des Spektrums.

11. Verfahren nach Anspruch 8, wobei das Erzeugen des dritten Bilds Folgendes umfasst:
Erfassen von Farbinformationen zu jedem Pixel des dritten Bilds und Bestimmen eines Hauttons für den einen Bereich des Körpers des Benutzers, der dem dritten Bild entspricht.

12. Verfahren nach Anspruch 8, ferner umfassend:
Ausgeben einer ersten Benutzerschnittstelle, die das erste Aufnahmebild oder ein Korrekturbild beinhaltet, in dem ein bestimmter Effekt dem ersten Aufnahmebild zugewiesen ist.

13. Verfahren nach Anspruch 12, wobei das Ausgeben der ersten Benutzerschnittstelle Folgendes umfasst:
wenn ein Benutzereingabeereignis auf einem Bereich des ersten Aufnahmebilds oder des Korrekturbilds erfolgt, Identifizieren eines Pixels, das einem Benutzereingabebereich auf dem ersten Aufnahmebild oder dem Korrekturbild entspricht, unter dem mindestens einen Pixel des dritten Bilds und Ausgeben von Hautinformationen zu einem Bereich des Körpers eines Benutzers, der dem identifizierten Pixel entspricht.

## Revendications

1. Dispositif électronique (100) comprenant :
un dispositif de capture d'image (120) comprenant un dispositif de source de lumière (121) configuré pour émettre un rayon infrarouge ; une première caméra (125) et une seconde caméra (127) configurée pour détecter le rayon infrarouge et pour capturer une image d'au moins une partie du corps d'un utilisateur sur la base du rayon infrarouge émis ;
une mémoire (130) configurée pour stocker une image capturée par le dispositif de capture d'image ;
un écran (150) configuré pour émettre une lumière dans une couleur spécifiée à partir d'au moins une zone sur la base du pilotage d'au moins un pixel ; et
au moins un processeur (140) branché électriquement au dispositif de capture d'image, à la mémoire et à l'écran, ledit au moins un processeur étant configuré pour :
commander afin d'amener l'écran à émettre la lumière dans la couleur spécifiée à partir de ladite au moins une zone,
commander afin de piloter le dispositif de capture d'image pour obtenir une première image de capture au moyen de la première caméra lorsque l'écran émet la lumière dans la couleur spécifiée et une seconde image de capture au moyen de la seconde caméra lorsque le dispositif de source de lumière émet le rayon infrarouge, générer une troisième image en effectuant l'enregistrement de la première image de capture et de la seconde image de capture, générer un spectre pour une lumière d'une bande de longueurs d'onde spécifique correspondant à un pixel spécifique de la troisième image générée, et déterminer un état cutané pour une zone du corps d'un utilisateur correspondant au pixel spécifique sur la base d'un gradient et d'une taille de la zone du spectre généré.

2. Dispositif électronique selon la revendication 1, ledit au moins un processeur étant en outre configuré pour :
construire une base de données pour une couleur d'image correspondant à une lumière d'une bande de longueurs d'onde spécifique, et déterminer une lumière d'une bande de longueurs d'onde spécifique correspondant à une couleur de chaque pixel de la troisième image en se référant à la base de données.

3. Dispositif électronique selon la revendication 2, ledit au moins un processeur étant en outre configuré pour :
déterminer un indice de mélanine pour la zone du corps de l'utilisateur sur la base du gradient du spectre, et déterminer un indice d'érythème pour la zone du corps de l'utilisateur sur la base de la taille de la zone du spectre.

4. Dispositif électronique selon la revendication 1, ledit au moins un processeur étant en outre configuré pour :
collecter des informations de couleur sur chaque pixel de la troisième image et déterminer un teint de peau pour la zone du corps de l'utilisateur correspondant à la troisième image.

5. Dispositif électronique selon la revendication 1, ledit au moins un processeur étant en outre configuré pour :
émettre une première interface utilisateur comprenant la première image de capture ou une image de correction dans laquelle un effet spécifié est attribué à la première image de capture.

6. Dispositif électronique selon la revendication 5, ledit au moins un processeur étant en outre configuré pour :
si un événement d'entrée d'utilisateur sur une zone de la première image de capture ou de l'image de correction se produit, identifier un pixel correspondant à une zone d'entrée d'utilisateur sur la première image de capture ou l'image de correction parmi au moins un pixel de la troisième image, et émettre des informations sur la peau sur une zone du corps d'un utilisateur correspondant au pixel identifié.

7. Dispositif électronique selon la revendication 1, ledit au moins un processeur étant en outre configuré pour :
si un événement d'activation d'un mode de capture d'image spécifié associé à la détermination d'un état cutané par capture d'image se produit, émettre une seconde interface utilisateur capable de régler une lumière d'au moins une bande de longueurs d'onde spécifique en relation avec l'émission de lumière de l'écran.

8. Procédé permettant de fournir des informations sur la peau dans un dispositif électronique, le procédé comprenant :
la capture (601) d'une image d'au moins une partie du corps d'un utilisateur sur la base d'un dispositif de source de lumière configuré pour émettre un rayon infrarouge, une première caméra et une seconde caméra configurée pour détecter le rayon infrarouge ;
l'émission (605) d'une lumière dans une couleur spécifiée à partir d'au moins une zone d'un écran du dispositif électronique sur la base du pilotage d'au moins un pixel de l'écran ;
la commande (607) afin de piloter le dispositif de capture d'image pour obtenir une première image de capture au moyen de la première caméra lorsque l'écran émet la lumière dans la couleur spécifiée et une seconde image de capture au moyen de la seconde caméra lorsque le dispositif de source de lumière émet le rayon infrarouge;
la génération (611) d'une troisième image en effectuant (609) un enregistrement de la première image de capture et de la seconde image de capture, la génération d'un spectre pour une lumière d'une bande de longueurs d'onde spécifique correspondant à un pixel spécifique de la troisième image générée, et la détermination d'un état cutané pour une zone du corps d'un utilisateur correspondant au pixel spécifique sur la base d'un gradient et d'une taille de la zone du spectre généré.

9. Procédé selon la revendication 8, comprenant en outre :
construire une base de données pour une couleur d'image correspondant à une lumière d'une bande de longueurs d'onde spécifique ; et
déterminer une lumière d'une bande de longueurs d'onde spécifique correspondant à une couleur de chaque pixel de la troisième image en se référant à la base de données.

10. Procédé selon la revendication 9, dans lequel la détermination de l'état cutané comprend :
la détermination d'un indice de mélanine pour ladite une zone du corps de l'utilisateur sur la base du gradient du spectre, et la détermination d'un indice d'érythème pour la zone du corps de l'utilisateur sur la base de la taille de la zone du spectre.

11. Procédé selon la revendication 8, ladite génération de la troisième image comprenant :
la collecter d'informations de couleur sur chaque pixel de la troisième image, et la détermination d'un teint de peau pour la zone du corps de l'utilisateur correspondant à la troisième image.

12. Procédé selon la revendication 8, comprenant en outre :
l'émission d'une première interface utilisateur comprenant la première image de capture ou une image de correction dans laquelle un effet spécifié est attribué à la première image de capture.

13. Procédé selon la revendication 12, ladite émission de la première interface utilisateur comprenant :
si un événement d'entrée d'utilisateur sur une zone de la première image de capture ou de l'image de correction se produit, l'identification d'un pixel correspondant à une zone d'entrée d'utilisateur sur la première image de capture ou l'image de correction parmi au moins un pixel de la troisième image, et l'émission d'informations sur la peau sur une zone du corps d'un utilisateur correspondant au pixel identifié.
